# EUROPEAN PATENT SPECIFICATION

(11) **EP 2 149 607 B1**
(45) Date of publication and mention of the grant of the patent: **20.12.2017**
(21) Application number: 08740187.3
(22) Date of filing: 10.04.2008
(51) Int. Cl.: C12P 7/46, C12P 7/62

(54) **METHOD FOR PRODUCTION OF SUCCINIC ACID**
VERFAHREN ZUR HERSTELLUNG VON SUCCINYLSÄURE
PROCÉDÉ DE PRODUCTION D'ACIDE SUCCINIQUE

(30) Priority: 10.04.2007 JP 2007102668
(43) Date of publication of application: 03.02.2010
(73) Proprietor: Ajinomoto Co., Inc., Chuo-ku, Tokyo 104-8315 (JP)
(72) Inventor: FUKUI, Keita, Kawasaki-shi Kanagawa 210-8681 (JP); MIHARA, Yoko, Kawasaki-shi Kanagawa 210-8681 (JP)
(74) Representative: Strehl Schübel-Hopf & Partner
(86) International application number: PCT/JP2008/057086
(87) International publication number: WO 2008/126896

(56) References cited:
- EP-A1- 1 672 077
- EP-A1- 1 947 190
- WO-A1-2007/046389
- JP-A- 2002 191 370
- JP-A- 2004 513 603
- JP-A- 2006 320 208
- SHOHEI OKINO ET AL: "Production of organic acids by Corynebacterium glutamicum under oxygen deprivation", APPLIED MICROBIOLOGY AND BIOTECHNOLOGY, SPRINGER, BERLIN, DE, vol. 68, no. 4, 1 September 2005 (2005-09-01), pages 475-480, XP019331935, ISSN: 1432-0614, DOI: 10.1007/S00253-005-1900-Y
- KEITA FUKUI ET AL: "Identification of succinate exporter in Corynebacterium glutamicum and its physiological roles under anaerobic conditions", JOURNAL OF BIOTECHNOLOGY, vol. 154, no. 1, 1 June 2011 (2011-06-01), pages 25-34, XP055005377, ISSN: 0168-1656, DOI: 10.1016/j.jbiotec.2011.03.010
- STEPHANIE HUHN ET AL: "Identification of the membrane protein SucE and its role in succinate transport in", APPLIED MICROBIOLOGY AND BIOTECHNOLOGY, SPRINGER, BERLIN, DE, vol. 89, no. 2, 31 August 2010 (2010-08-31), pages 327-335, XP019870362, ISSN: 1432-0614, DOI: 10.1007/S00253-010-2855-1
- WANG Q. ET AL.: 'Expression of galactose permease and pyruvate carboxylase in Escherichia coli ptsG mutant increases the growth rate and succinate yield under anaerobic conditions' BIOTECHNOL. LETT. vol. 28, 2006, pages 89 - 93, XP019231182
- IKEDA M. ET AL.: 'the Corynebacterium glutamicum genome: features and impacts on biotechnological processes' APPL. MICROBIOL. BIOTECHNOL. vol. 62, 2003, pages 99 - 109, XP002419971

## Description

### Technical Field

The present invention relates to a method for producing succinic acid using a bacterium such as coryneform bacteria.

### Background Art

For the production of non-amino organic acids including succinic acid by fermentation, anaerobic bacteria including those belonging to the genus *Anaerobiospirillum* or *Actinobacillus* are usually used (Patent documents 1 and 2, Non-patent document 1). Although use of such anaerobic bacteria provides high yields of products, many nutrients are required for their proliferation, and therefore it is necessary to add large amounts of organic nitrogen sources such as corn steep liquor (CSL) into culture medium. Addition of large amounts of organic nitrogen sources results in not only increase in cost for culture medium, but also increase in purification cost for isolating the product, and therefore it is not economical.

In addition, methods in which aerobic bacteria such as coryneform bacteria are once cultured under aerobic conditions to proliferate bacterial cells, then harvested, washed, and allowed as resting cells to produce a non-amino organic acid without supplying oxygen (Patent documents 3 and 4) are also known. When aerobic bacteria are used for the production of non-amino organic acids as described above, culture under microaerobic conditions (microaerobic induction) is generally needed for imparting an activity for producing succinic acid under anaerobic conditions to the cells proliferated under aerobic conditions. These methods are economical, since organic nitrogen may be added in a smaller amount for proliferating the bacterial cells, and the bacteria can sufficiently grow in a simple culture medium. However, there is still a room for improvement in terms of production amount, concentration, and production rate per cell of the target organic acids as well as simplification of production process, and the like. Furthermore, techniques of increasing a non-amino organic acid-producing ability by DNA recombination are also disclosed. For example, production of a non-amino organic acid by fermentation using a bacterium in which phosphoenolpyruvate carboxylase activity is enhanced (for example, Patent document 5), and the like have also been reported.

Entire genome sequence of a coryneform bacterium has been elucidated, and functions of putative protein-coding sequences are predicted (Non-patent document 2). The gene called *sucE1* in the present invention is one of them, and although it is expected to code for a permease, the actual function thereof has not been clarified, and participation thereof in the succinic acid synthetic pathway was not known at all, either. As for maleate dehydrogenase, although a method for producing succinic acid using a bacterium in which activity of this enzyme is enhanced has been reported (Patent document 6), effect of enhancing the *mdh* gene coding for that enzyme along with the *sucE1* gene is not known to date.
Patent document 1: U.S. Patent No. 5,142,834
Patent document 2: U.S. Patent No. 5,504,004
Patent document 3: Japanese Patent Laid-open (KOKAI) No. 11-113588
Patent document 4: Japanese Patent Laid-open No. 11-196888
Patent document 5: Japanese Patent Laid-open No. 11-196887
Patent document 6: Japanese Patent Laid-open No. 2006-320208
Non-patent document 1: International Journal of Systematic Bacteriology, 49, 207-216 (1999)
Non-patent document 2: Appl. Microbiol. Biotechnol., 62 (2-3), 99-109 (2003)

### Disclosure of the Invention

### Object to be Achieved by the Invention

An object of the present invention is to provide a method for producing succinic acid using a bacterium, of which production process is simplified.

### Means for Achieving the Object

The inventors of the present invention conducted various researches in order to achieve the aforementioned object, as a result, found that a bacterium which has been modified so that expressions of *sucE1* gene and *mdh* gene are enhanced can produce succinic acid from an organic raw material without need of microaerobic induction, and thus accomplished the present invention.

That is, the present invention relates to the followings.
[1] A method for producing succinic acid, which comprises allowing an aerobically cultured bacterium or cells thereof, which have been immobilized or disrupted, or a supernatant of said disrupted cells, or a partially purified fraction of said supernatant, wherein the bacterium has an ability to produce succinic acid and has been modified so that expressions of *sucE1* gene encoding succinate exporter and *mdh* gene encoding malate dehydrogenase are enhanced to act on an organic raw material in a reaction mixture containing carbonate ions, bicarbonate ions, or carbon dioxide gas to produce the succinic acid, and collecting the succinic acid.
[2] The method according to [1], wherein the bacterium has been modified so that expression amounts of the *sucE1* gene and the *mdh* gene under aerobic conditions are increased by 1.5 times or more, respectively, as compared to an unmodified strain.
[3] The method according to [1] or [2], wherein the bacterium is a bacterium selected from the group consisting of coryneform bacteria, *Bacillus* bacteria, and *Rhizobium* bacteria.
[4] The method according to any one of [1] to [3], wherein expressions of the *sucE1* gene and/or the *mdh* gene is enhanced by increasing copy number of the *sucE1* gene and/or the *mdh* gene, by modifying an expression control sequence of these genes, or by replacing a promoter of these genes with a stronger promoter.
[5] The method according to [4], wherein the stronger promoter is a constitutive expression promoter.
[6] The method according to [4] or [5], wherein the stronger promoter is a promoter of a gene selected from genes coding for elongation factor Tu, cochaperonin GroES-chaperonin GroEL, thioredoxin reductase, phosphoglycerate mutase, peroxiredoxin, glycerol-3-phosphate dehydrogenase, 2,3-butanediol dehydrogenase, fructose bisphosphate aldolase, and superoxide dismutase.
[7] The method according to any one of [1] to [6], wherein the *sucE1* gene is a DNA defined in (a) or (b):
   (a) a DNA comprising the nucleotide sequence of the nucleotide numbers 571 to 2187 of SEQ ID NO: 3,
   (b) a DNA which hybridizes with a nucleotide sequence complementary to the nucleotide sequence of the nucleotide numbers 571 to 2187 of SEQ ID NO: 3 under stringent conditions, which improves succinic acid-producing ability of the bacterium when expression thereof is enhanced in the bacterium.
[8] The method according to any one of [1] to [7], wherein the *mdh* gene is a DNA defined in (c) or (d):
   (a) a DNA comprising the nucleotide sequence of the nucleotide numbers 301 to 1287 of SEQ ID NO: 13,
   (b) a DNA which hybridizes with a nucleotide sequence complementary to the nucleotide sequence of the nucleotide numbers 301 to 1287 of SEQ ID NO: 13 under stringent conditions, and codes for a protein having malate dehydrogenase activity.
[9] The method according to any one of [1] to [8], wherein the bacterium has been further modified so that lactate dehydrogenase activity is decreased to 10% or less of the activity of an unmodified strain.
[10] The method according to any one of [1] to [9], wherein the bacterium has been further modified so that pyruvate carboxylase activity is enhanced.
[11] A method for producing a succinic acid-containing polymer, which comprises the steps of:
   producing succinic acid by the method according to any one of [1] to [10], and
   polymerizing the obtained succinic acid.
[12] The method according to any one of [1] to [10], wherein the bacterium has been further modified so that succinate dehydrogenase activity is enhanced.

### Brief Description of the Drawings

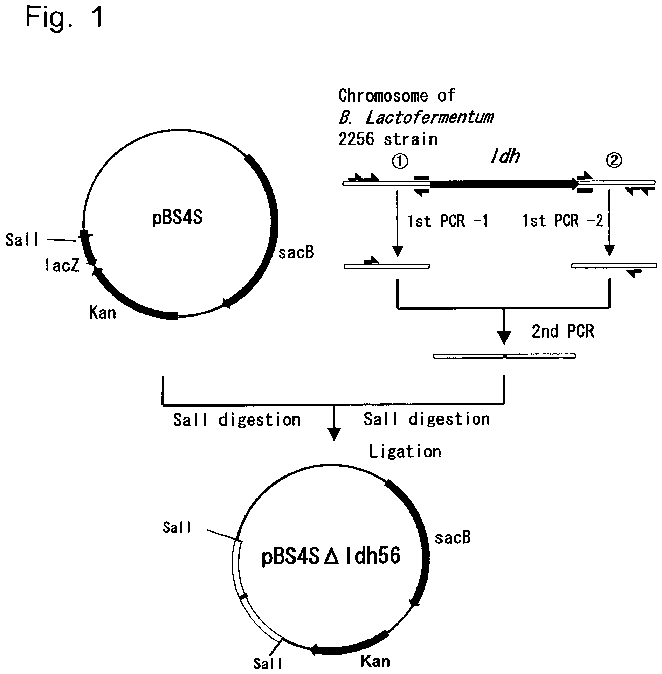

Fig. 1 shows construction of plasmid pBS4SΔldh5 for *ldh* gene disruption.

### Best Mode for Carrying out the Invention

Hereinafter, embodiments of the present invention will be explained in detail.

### Bacteria used for the present invention

The bacterium used for the method of the present invention (henceforth also referred to as "the bacterium of the present invention") is a bacterium which has an ability to produce succinic acid and has been modified so that expressions of the *sucE1* gene and the *mdh* gene are enhanced. Such a bacterium can be obtained by modifying a bacterium having succinic acid-producing ability as a parent strain so that expressions of the *sucE1* gene and the *mdh* gene in the bacterium are simultaneously enhanced. When a parent strain does not have the succinic acid-producing ability, such a bacterium can be obtained by imparting succinic acid-producing ability to the parent strain and modifying the strain so that expressions of the *sucE1* gene and the *mdh* gene are enhanced. Furthermore, such a bacterium can also be obtained by imparting succinic acid-producing ability to a strain which has been modified so that expressions of the *sucE1* gene and the *mdh* gene are enhanced.

Parent strains of the bacteria that can be used in the present invention are not particularly limited. However, aerobic bacteria and facultative anaerobic bacteria are preferred, specifically, coryneform bacteria, *Bacillus* bacteria, *Rhizobium* bacteria, and *Escherichia* bacteria are preferred, and among these, coryneform bacteria are more preferred. Examples of the coryneform bacteria include microorganisms belonging to the genus *Corynebacterium,* microorganisms belonging to the genus *Brevibacterium,* and microorganisms belonging to the genus *Arthrobacter.* Among these, those belonging to the genus *Corynebacterium* or *Brevibacterium* are more preferred. Still more preferred are microorganisms belonging to *Corynebacterium glutamicum, Brevibacterium flavum, Brevibacterium ammoniagenes,* or *Brevibacterium lactofermentum.*

Particularly preferred specific examples of the aforementioned parent strains of bacteria include *Brevibacterium flavum* MJ-233 (Agric. Biol. Chem., 54 (2), 443-447, 1990), MJ-233 AB-41, which is a mutant strain of MJ-233 (Japanese Patent Laid-open No. 2003-235592), *Corynebacterium glutamicum (Corynebacterium* sp., *Brevibacterium flavum*) AJ110655 (FERM ABP-10951), *Brevibacterium ammoniagenes* ATCC 6872, *Corynebacterium glutamicum* ATCC 31831, ATCC 13032, *Brevibacterium lactofermentum* ATCC 13869, and the like. Since *Brevibacterium flavum* may be currently classified into *Corynebacterium glutamicum* (Lielbl, W., Ehrmann, M., Ludwig, W. and Schleifer, K.H., International Journal of Systematic Bacteriology, 1991, vol. 41, pp.255-260), the *Brevibacterium flavum* MJ-233 strain, MJ-233 AB-41 strain, and AJ110655 strain are considered to be identical to the *Corynebacterium glutamicum* MJ-233 strain, MJ-233 AB-41 strain, and AJ110655 strain, respectively, in the present invention.

The *Corynebacterium glutamicum* AJ110655 strain was deposited at the International Patent Organism Depository, Agency of Industrial Science and Technology (Central 6, 1-1, Higashi 1-chome, Tsukuba-shi, Ibaraki-ken, Japan, 305-8566) on February 15, 2008 and assigned an receipt number of FERM ABP-10951. The *Corynebacterium glutamicum* AJ110655 is an LDH gene-deficient strain constructed from the *Brevibacterium flavum* MJ-233 strain, and was deposited as the *Corynebacterium glutamicum* AJ110655 strain. Then, it was suggested that the species thereof was different from *Corynebacterium glutamicum* on the basis of nucleotide sequence analysis of 16S rRNA etc.

The bacteria to be used as a parent strain for obtaining the bacterium used in the method of the present invention may be, besides wild-type strains, any of strains including mutant strains obtained by usual mutation treatments such as UV irradiation and NTG treatment, and recombinant strains induced by genetic procedures such as cell fusion and gene recombination techniques.

When the parent strain does not have succinic acid-producing ability, the succinic acid-producing ability is imparted by a mutation treatment or gene recombination. However, when succinic acid-producing ability is imparted by modification to enhance expressions of the *sucE1* gene and the *mdh* gene, it is not always necessary to impart the succinic acid-producing ability by another means.

The "succinic acid-producing ability" used in the present invention refers to an ability of the bacterium to accumulate succinic acid in a reaction mixture containing carbonate ions, bicarbonate ions, or carbon dioxide to such an extent that the succinic acid can be collected from the reaction mixture when the bacterium or a product obtained by processing the bacterium is allowed to act on a organic raw material in the reaction mixture.

The methods of imparting succinic acid-producing ability to a microorganism will be specifically explained below.

### Succinic acid-producing bacteria

Examples of the method for imparting or enhancing succinic acid-producing ability by breeding include, for example, modifying a bacterium so that expression of a gene coding for an enzyme involved in succinic acid biosynthesis is enhanced. Examples of the enzyme involved in succinic acid biosynthesis include, for example, pyruvate carboxylase as described later, fumarate reductase described in Japanese Patent Laid-open No. 2005-095169, and the like. Bacteria in which the pyruvate carboxylase and fumarate reductase genes are amplified are described in Japanese Patent Laid-open based on International Patent Application (Kohyo) No. 2002-511250, Japanese Patent Laid-open Nos. 11-196888, 2005-95169, and the like.

Succinic acid-producing ability can also be imparted or enhanced by disrupting a gene coding for lactate dehydrogenase, which is an enzyme that is expressed under anaerobic conditions as described later. Succinic acid-producing ability can also be imparted by treating a bacterium with ultraviolet ray or with a mutagen used in usual mutation treatments such as N-methyl-N'-nitro-N-nitrosoguanidine (NTG) or ethyl methanesulfonate (EMS) and selecting a strain which produces succinic acid. Examples of mutant having succinic acid-producing ability include the glutamic acid-auxotrophic strain disclosed in Japanese Patent Laid-open No. 2005-065641, and the like.

The bacterium used for the method of the present invention can be obtained by modifying such a bacterium having succinic acid-producing ability as described above so that expressions of the *sucE1* gene and the *mdh* gene are enhanced. Either the modification for imparting succinic acid-producing ability or the modification for enhancing expressions of the *sucE1* gene and the *mdh* gene may be performed first.

### sucE1 gene

The protein encoded by the *sucE1* gene (SucE, succinate exporter) is presumed to be a kind of permease, and is a protein which improves succinic acid-producing ability of a bacterium when expression of the gene is enhanced in the bacterium.

Examples of the *sucE1* gene include the *sucE1* gene derived from *Brevibacterium flavum* MJ-233 (nucleotide numbers 571 to 2187 of SEQ ID NO: 3), the *sucE1* gene derived from *Corynebacterium glutamicum* ATCC 13032 (gene having the nucleotide sequence of SEQ ID NO: 5), the *sucE1* gene derived from *Corynebacterium efficiens* YS314 (gene having the nucleotide sequence of SEQ ID NO: 7), the *sucE1* gene derived from *Corynebacterium diphtheriae gravis* NCTC 13129 (gene having the nucleotide sequence of SEQ ID NO: 11), and the like. The *sucE1* gene derived from C. *glutamicum* ATCC 13032 is registered as NCg12130 in the genome sequence registered as GenBank Accession No. NC_003450 (amino acid sequence is resistered as GenBank Accession No. NP_601414). The *sucE1* gene derived from C. *efficiens* YS314 is resistered as CE2102 in the genome sequence resistered as GenBank Accession No. NC_004369. The *sucE1* gene derived from C. *diphtheriae gravis* NCTC 13129 is resistered as DIP0830 of GenBank Accession No. NC_002935.

As the *sucE1* gene, a homologue gene of *sucE1* derived from another microorganism may be used so long as a gene which can improve succinic acid-producing ability of a bacterium when expression thereof is enhanced in the bacterium is used. Homologues of the *sucE1* gene may be searched for by using BLAST (//blast.genome.jp/) or the like by referring to the sequence of the nucleotide numbers 571 to 2187 of SEQ ID NO: 3, and the like.

Since several sequences of the *sucE1* gene have already been determined, the gene can be obtained by PCR using primers prepared on the basis of the nucleotide sequences. For example, a region including the structural *sucE1* gene of *C*. *glutamicum* and a flanking region thereof including a control region of the gene can be obtained by PCR (polymerase chain reaction, see White, T.J. et al., Trends Genet., 5, 185 (1989)) using the primers shown in SEQ ID NOS: 1 and 2 and chromosomal DNA of a coryneform bacterium as the template. Homologues of *sucE1* of other microorganisms can also be obtained in a similar manner.

Since the nucleotide sequence of the *sucE1* gene is different depending on species or strains of coryneform bacteria, the *sucE1* gene to be used for obtaining a bacterium used in the method of the present invention is not limited to a gene having the sequence of the nucleotide numbers 571 to 2187 of SEQ ID NO: 3 or the sequence of SEQ ID NO: 5, 7 or 11, and it may be a mutant or artificially modified gene that codes for a protein having a sequence of SEQ ID NO: 4, 6, 8 or 12 including substitutions, deletions, insertions, additions, etc. of one or several amino acid residues at one or more positions so long as it remains to be a gene that improves succinic acid-producing ability of a bacterium when expression of the gene is enhanced in the bacterium. Although number meant by the term "several" used herein may differ depending on positions in the three-dimensional structure of the protein or types of amino acid residues, it may be specifically 2 to 20, preferably 2 to 10, more preferably 2 to 5. The substitutions, deletions, insertions, additions, inversions or the like of amino acid residues described above also includes those caused by a naturally occurring mutation based on individual difference, difference in species of microorganisms that contain the *sucE1* gene (mutant or variant), or the like.

The aforementioned substitution is preferably a conservative substitution that is a neutral substitution not providing functional change. The conservative mutation is a mutation wherein substitution takes place mutually among Phe, Trp and Tyr, if the substitution site is an aromatic amino acid; among Leu, Ile and Val, if the substitution site is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having hydroxyl group. Specific examples of substitution considered conservative substitution include: substitution of Ser or Thr for Ala; substitution of Gln, His or Lys for Arg; substitution of Glu, Gln, Lys, His or Asp for Asn; substitution of Asn, Glu or Gln for Asp; substitution of Ser or Ala for Cys; substitution of Asn, Glu, Lys, His, Asp or Arg for Gln; substitution of Gly, Asn, Gln, Lys or Asp for Glu; substitution of Pro for Gly; substitution of Asn, Lys, Gln, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution of Trp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val.

Furthermore, as the *sucE1* gene, there may be used a sequence encoding a protein showing a homology not less than 80%, preferably not less than 90%, more preferably not less than 95%, particularly preferably not less than 97%, to the entire amino acid sequence of SEQ ID NOS: 4, 6, 8 or 12 and coding for a protein which improves succinic acid-producing ability of a bacterium when expression thereof is enhanced in the bacterium. Furthermore, degree of degeneracy of a gene varies depending on hosts into which the gene is introduced, and therefore codons may be replaced with those which are easily used by a host into which *sucE1* is introduced. Moreover, the *sucE1* gene may be a gene coding for a protein of which N- or C-terminal sequence is elongated or deleted, so long as the gene has a function for improving succinic acid-producing ability of a bacterium when expression thereof is enhanced in the bacterium. The length of amino acid sequence to be elongated or deleted may be 50 or less, preferably 20 or less, more preferably 10 or less, particularly preferably 5 or less, in terms of number of amino acid residues. More specifically, the *sucE1* gene may be a gene coding for a protein having the amino acid sequence of SEQ ID NO: 4, 6, 8 or 12 with elongation or deletion of 5 to 50 amino acid residues on the N-terminal side or 5 to 50 amino acid residues on the C-terminal side.

Such genes homologous to the *sucE1* gene as described above can be obtained by modifying the nucleotide sequence of nucleotide numbers 571 to 2187 of SEQ ID NO: 3, or the nucleotide sequence of SEQ ID NO: 5, 7 or 11 so that the protein encoded by the gene include substitutions, deletions, insertions, or additions of amino acid residues at a specific site(s) by, for example, site-specific mutagenesis. Furthermore, such genes can also be obtained by conventionally known mutation treatment described below. Examples of the mutation treatment include treating the nucleotide sequence of nucleotide numbers 571 to 2187 of SEQ ID NO: 3, or the nucleotide sequence of SEQ ID NO: 5, 7 or 11 with hydroxylamine or the like *in vitro,* and treating a microorganism, for example, coryneform bacterium, containing the gene with ultraviolet ray irradiation or a mutagen used in a usual mutation treatment such as N-methyl-N'-nitro-N-nitrosoguanidine (NTG) or ethyl methanesulfonate (EMS). Furthermore, a mutation can be artificially introduced into the *sucE1* gene by gene recombination using error-prone PCR, DNA shuffling, or StEP-PCR to obtain a highly active *sucE1* gene (Firth A.E., Patrick W.M., Bioinformatics., 21, 3314-3315, 2005 Jun. 2; Statistics of protein library construction).

Whether such *sucE1* homologue genes code for a protein which improves succinic acid-producing ability when expression thereof is enhanced can be confirmed by, for example, introducing these genes into a wild-type strain of a bacterium and examining whether succinic acid-producing ability of the bacterium is improved or not.

Examples of the *sucE1* gene also include a DNA that hybridizes with a sequence complementary to the sequence of nucleotide numbers 571 to 2187 of SEQ ID NO: 3 or the nucleotide sequence of SEQ ID NO: 5, 7 or 11, or a probe that can be prepared from the sequences under stringent conditions and codes for a protein which improves succinic acid-producing ability of a bacterium when expression thereof is enhanced in the bacterium. The "stringent conditions" referred to herein is a condition under which a so-called specific hybrid is formed, and non-specific hybrid is not formed. Examples include, for example, conditions where DNAs showing high homology to each other, for example, DNAs showing a homology of, for example, not less than 80%, preferably not less than 90%, more preferably not less than 95%, particularly preferably not less than 97%, hybridize with each other, and DNAs having homology lower than the above level do not hybridize with each other, and conditions of washing in ordinary Southern hybridization, i.e., conditions of washing once, preferably twice or three times, at salt concentrations and temperature of 1 x SSC, 0.1% SDS at 60°C, preferably 0.1 x SSC, 0.1% SDS at 60°C, more preferably 0.1 x SSC, 0.1% SDS at 68°C.

A partial sequence of the sequence of the nucleotide numbers 571 to 2187 of SEQ ID NO: 3 or the sequence of SEQ ID NO: 5, 7 or 11 may also be used as the probe. Such a probe may be prepared by PCR using oligonucleotides prepared on the basis of these nucleotide sequences as primers and a DNA fragment containing the sequence as the template. When a DNA fragment having a length of about 300 bp is used as the probe, the washing condition after hybridization under the aforementioned condition can be exemplified by 2 x SSC, 0.1% SDS at 50°C.

The expression "modified so that expression of *sucE1* gene is enhanced" means that the number of SucE1 product molecules per cell is increased, that the activity per SucE1 product molecule is increased, etc., as compared to an unmodified strain such as a parent strain or a wild-type strain. Examples of the wild-type coryneform bacterium to be used for comparison include *Corynebacterium glutamicum* (*Brevibacterium lactofermentum*) ATCC 13869, ATCC 13032, and the like (the same shall apply to the other genes mentioned below).

Increase of expression level of the *sucE1* gene can be confirmed by comparing level of mRNA of *sucE1* with that of an unmodified strain such as a parent strain or a wild-type strain. Examples of the method for confirming the expression level include Northern hybridization and RT-PCR (Molecular Cloning (Cold Spring Harbor Laboratory Press, Cold Spring Harbor (USA), 2001). Expression level of *sucE1* may be any level so long as the level is increased as compared to that of an unmodified strain, and for example, it is desirably increased not less than 1.5 times, more preferably not less than 2 times, further preferably not less than 3 times, as compared to that of an unmodified strain. In the present invention, the "aerobic conditions" means that dissolved oxygen concentration is not lower than 0.33 ppm and not higher than 7 ppm, more preferably not lower than 1.5 ppm and not higher than 7 ppm (Amino Acid Fermentation, Kunihiko Akashi et al.).

### mdh gene

The *mdh* gene is not particularly limited so long as a gene coding for a protein having malate dehydrogenase activity is used, and examples include, for example, the gene derived from the *Brevibacterium flavum* MJ-233 strain having the nucleotide sequence shown in SEQ ID NO: 13. The "malate dehydrogenase activity" referred to herein means the activity for catalyzing the reaction of reducing oxaloacetic acid to convert it into malic acid, and the expression "malate dehydrogenase activity is enhanced" means that the malate dehydrogenase activity is enhanced as compared to that of a malate dehydrogenase-unmodified strain.

As the *mdh* gene, a homologue gene of *mdh* derived from another microorganism may be used so long as the gene codes for a protein having the malate dehydrogenase activity. Homologues of the *mdh* gene may be searched for by using BLAST (http://blast.genome.jp/) or the like with referring to the sequence of the nucleotide numbers 301 to 1384 of SEQ ID NO: 13.

Since several sequences of the *mdh* gene have already been determined as described above, the gene can be obtained by PCR using primers prepared on the basis of the nucleotide sequences. For example, a region including the structural *mdh* gene of *C. glutamicum* and a flanking region thereof including a control region of the gene can be obtained by PCR using the primers shown in SEQ ID NOS: 15 and 16 and chromosomal DNA of the coryneform bacterium as the template. Homologues of *mdh* of other microorganisms can also be obtained in a similar manner.

The foregoing descriptions concerning mutants or artificially modified types of the *sucE1* and protein encoded thereby are also applied to the *mdh* gene. These descriptions are also applied to the LDH gene and PC gene described later.

The expression "modified so that expression of *mdh* gene is enhanced" means that the bacterium is modified so that the activity of malate dehydrogenase encoded by the mdh gene is enhanced as compared to an *mdh* unmodified strain such as a parent strain or a wild-type strain, and such a state includes a state that the number of malate dehydrogenase molecules per cell is increased, a state that the activity per maleate dehydrogenase molecule is increased, etc. Examples of the wild-type coryneform bacterium to be used for comparison include *Corynebacterium glutamicum* (*Brevibacterium lactofermentum*) ATCC 13869, ATCC 13032, and the like.

Increase of expression level of the *mdh* gene can be confirmed by comparing level of mRNA of *mdh* with that of an unmodified strain as described above. Increase of expression level of the *mdh* gene can also be confirmed by comparing the maleate dehydrogenase activity with that of an unmodified strain.

Expression level of the *mdh* gene may be increased to any level so long as the level is increased as compared to that of an unmodified strain under aerobic conditions, and for example, it is desirably increased not less than 1.5 times, more preferably not less than 2 times, as compared to an unmodified strain. The malate dehydrogenase activity can be measured by the method of measuring decrease of NADH as described later.

### Enhancement of expressions of sucE1 gene and mdh gene

Expressions of the *sucE1* gene and the *mdh* gene may be enhanced by increasing the copy numbers of these genes. For example, the copy numbers of the genes may be increased by ligating a fragment containing the genes to a vector which functions in a bacterium, preferably a multi copy vector, to prepare a recombinant DNA, and transforming such a bacterium as described above with the DNA. Alternatively, the copy numbers of the genes may be increased by transferring one copy or multiple copies of the genes to a chromosome. Transfer of the genes to the chromosome can be confirmed by Southern hybridization using a part of the genes as a probe.

When a plasmid vector is used, the *sucE1* gene and the *mdh* gene may be carried by the same plasmid, or may be separately carried by different plasmids. Moreover, the order of the introductions of the *sucE1* gene and the *mdh* gene into a bacterium is not limited, and they may be introduced simultaneously.

Expressions of the *sucE1* gene and the *mdh* gene can also be enhanced by modifying an expression control sequence of these genes. For example, the enhancement can be achieved by replacing a promoter sequence of the genes with a stronger promoter, or by making a promoter sequence closer to a consensus sequence (WO00/18935).

Methods for modifying a microorganism so that expressions of the *sucE1* gene and the *mdh* gene are enhanced are specifically explained below. These methods can be performed as described in a manual such as Molecular Cloning (Cold Spring Harbor Laboratory Press, Cold Spring Harbor (USA), 2001).

First, a target gene is cloned from a chromosome of a coryneform bacterium or the like. A chromosomal DNA can be prepared from a bacterium serving as a DNA donor by, for example, the method of Saito and Miura (see H. Saito and K. Miura, Biochem. Biophys. Acta, 72, 619 (1963), Text for Bioengineering Experiments, Edited by the Society for Bioscience and Bioengineering, Japan, p 97-98, Baifukan Co., Ltd., 1992) or the like. Oligonucleotides used in PCR can be synthesized on the basis of the aforementioned known information, for example, the synthetic oligonucleotides shown ins SEQ ID NOS: 1 and 2 can be used to amplify the *sucE1* gene, and the synthetic oligonucleotides shown in SEQ ID NOS: 15 and 16 can be used to amplify the *mdh* gene.

A gene fragment comprising a gene amplified by PCR can be amplified by inserting the fragment into a vector having a replication origin that enables autonomous replication in a bacterium into which the vector is introduced and transforming the bacterium with the vector. In particular, in the case of introducing the fragment into a coryneform bacterium, if a recombinant DNA is prepared by ligating the fragment to a vector DNA that is autonomously replicable in cells of *Escherichia coli* and a coryneform bacterium, and introduced into *Escherichia coli,* subsequent operations becomes easier. Examples of the vector that is autonomously replicable in cells of *Escherichia coli* include pUC19, pUC18, pHSG299, pHSG399, pHSG398, RSF1010, pBR322, pACYC184, pMW219, and the like.

Examples of the plasmid that is autonomously replicable in coryneform bacteria include plasmid pCRY30 described in Japanese Patent Laid-open No. 3-210184; plasmids pCRY21, pCRY2KE, pCRY2KX, pCRY31, pCRY3KE, and pCRY3KX described in Japanese Patent Laid-open No. 2-72876 and U.S. Patent No. 5,185,262; plasmids pCRY2 and pCRY3 described in Japanese Patent Laid-open No. 1-191686; pAM330 described in Japanese Patent Laid-open No. 58-67679; pHM1519 described in Japanese Patent Laid-open No. 58-77895; pAJ655, pAJ611, and pAJ1844 described in Japanese Patent Laid-open No. 58-192900; pCG1 described in Japanese Patent Laid-open No. 57-134500; pCG2 described in Japanese Patent Laid-open No. 58-35197; pCG4, pCG11 etc. described in Japanese Patent Laid-open No. 57-183799; and pVK7 described in Japanese Patent Laid-open No. 10-215883.

Furthermore, a vector obtained by excising a DNA fragment which enables a plasmid to autonomously replicate in coryneform bacteria from any of those vectors and inserting the fragment into any of the aforementioned vectors for *Escherichia coli* can be used as a so-called shuttle vector which is autonomously replicable both in *Escherichia coli* and coryneform bacteria.

To prepare a recombinant DNA by ligation of the *sucE1* gene and the *mdh* gene to a vector that functions in coryneform bacteria, the vector is digested with a restriction enzyme suitable for the ends of the genes. Such a restriction enzyme site may be introduced in advance into a synthetic oligonucleotide to be used for amplifying the genes. Ligation is usually performed by using a ligase such as T4 DNA ligase.

In order to introduce a recombinant plasmid prepared as described above into a bacterium, any known transformation method reported to date can be employed. For example, there are a method of treating recipient cells with calcium chloride so as to increase permeability for DNA, which has been reported for *Escherichia coli* K-12 (Mandel, M. and Higa, A., J. Mol. Biol., 53, 159 (1970)), and a method of using competent cells prepared from growing cells and introducing DNA into them, which has been reported for *Bacillus subtilis* (Duncan, C. H., Wilson, G. A. and Young, F. E., Gene, 1, 153 (1977)). Also employable is a method of making DNA recipient cells into protoplasts or spheroplasts which easily take up a recombinant DNA, and introducing a recombinant DNA into them, which are known for *Bacillus subtilis,* actinomycetes, and yeasts (Chang, S. and Choen, S.N., Mol. Gen. Genet., 168, 111 (1979); Bibb, M.J., Ward, J.M. and Hopwood, O.A., Nature, 274, 398 (1978); Hinnen, A., Hicks, J.B. and Fink, G.R., Proc. Natl. Acad. Sci. USA, 75, 1929 (1978)). In addition, transformation of bacteria can also be performed by the electric pulse method (Japanese Patent Laid-open No. 2-207791) or by the conjugal transfer method (Biotechnology (NY). 1991 January; 9(1):84-7).

Copy numbers of the *sucE1* and *mdh* genes can also be increased by integrating multiple copies of the genes into a chromosomal DNA of a bacterium. In order to integrate multiple copies of the genes into a chromosomal DNA of a bacterium, homologous recombination is performed by targeting a sequence which exists in multiple copies on a chromosomal DNA. As the sequence present on a chromosomal DNA in a multiple copy number, a repetitive DNA or inverted repeat present at the end of a transposable element can be used. Alternatively, as disclosed in Japanese Patent Laid-open No. 2-109985, multiple copies of the genes can be introduced into a chromosomal DNA by incorporating them into a transposon and transferring it (Japanese Patent Laid-open Nos. 2-109985, 7-107976, Mol. Gen. Genet., 245, 397-405 (1994); Plasmid, 2000 November; 44(3): 285-91).

Also conceivable is a method of inserting the *sucE1* gene and/or the *mdh* gene into a plasmid having a replication origin that is not replicable in a host, or a plasmid having a replication origin that cannot replicate in a host and an ability to cause conjugal transfer to a host, and introducing the obtained plasmid into the host to amplify the gene on a chromosome. Examples of such a plasmid include pSUP301 (Simo et al., Bio/Technology 1, 784-791 (1983)), pK18mob or pK19mob (Schaefer et al., Gene 145, 69-73 (1994)), pGEM-T (Promega corporation, Madison, Wis., USA), pCR2.1-TOPO (Shuman (1994) Journal of Biological Chemistry 269: 32678-84; U.S. Patent No. 5,487,993), pCR^{(R)} Blunt (Invitrogen, Groningen, Netherlands; Bernard et al., Journal of Molecular Biology, 234: 534-541 (1993)), pEM1 (Schrumpf et al., 1991, Journal of Bacteriology 173: 4510-4516), pBGS8 (Spratt et al., 1986, Gene, 41:337-342), and the like. A plasmid vector comprising the *sucE1* gene and/or the *mdh* gene is transferred into a bacterium by conjugation or transformation to transfer the genes into a chromosome. The conjugation method is described by, for example, Schaefer et al. (Applied and Environmental Microbiology, 60, 756-759 (1994)). The transformation method is described by, for example, Theirbach et al. (Applied Microbiology and Biotechnology 29, 356-362 (1988)), Dunican and Shivinan (Bio/Technology 7, 1067-1070 (1989)), and Tauch et al. (FEMS Microbiological Letters 123, 343-347 (1994)).

Expressions of *sucE1* and *mdh* can also be enhanced by replacing expression control sequences of *sucE1* and *mdh* on a chromosomal DNA or a plasmid such as promoters with stronger promoters. For example, *lac* promoter, *trp* promoter, *trc* promoter, PS2 promoter, and the like are known as strong promoters. Methods for evaluating strength of promoters and examples of strong promoters are described in the paper of Goldstein et al. (Prokaryotic promoters in Biotechnology, Biotechnol. Annu. Rev., 1995, 1, 105-128), and the like. The strong promoter is more preferably a constitutive expression promoter. The constitutive expression promoter referred to here means a promoter of which expression is not changed by microaerobic induction. The microaerobic induction referred to here corresponds to culture under microaerobic conditions for imparting an activity for producing an organic acid under anaerobic conditions to cells proliferated under aerobic conditions. The microaerobic conditions corresponds to 2.ppm or lower, preferably 1 ppm or lower, more preferably 0.5 ppm or lower, in terms of dissolved oxygen concentration with or without aeration. Moreover, the anaerobic condition is a condition of dissolved oxygen concentration of 0.5 ppm or lower with bubbling of a gas other than oxygen such as nitrogen or carbon dioxide.

The expression "expression is not changed by microaerobic induction" means that the change ratio of the promoter activities observed before and after the microaerobic induction is 3 times or smaller, preferably 2 times or smaller, more preferably 1.5 times or smaller. Specific examples of the constitutive expression promoter include promoters of genes coding for the elongation factor Tu (EF-Tu) (SEQ ID NO: 45), cochaperonin GroES-chaperonin GroEL (SEQ ID NO: 39), thioredoxin reductase (SEQ ID NO: 43), phosphoglycerate mutase (SEQ ID NO: 38), peroxiredoxin (SEQ ID NO: 40), glyceraldehyde 3-phosphate dehydrogenase (SEQ ID NO: 42), L-2,3-butanediol dehydrogenase (SEQ ID NO: 41), fructose bisphosphate aldolase (SEQ ID NO: 44), superoxide dismutase (SEQ ID NO: 37), and the like, but not limited to these (WO2006/028063, European Patent No. 1697525).

The substitution of a stronger promoter may be combined with increasing copy numbers of the *sucE1* and *mdh* genes.

Expressions of *sucE1* and *mdh* can also be enhanced by modifying a factor involved in expression control of these genes such as an operator or a repressor, or ligating a strong terminator (Hamilton et al., Journal of Bacteriology 171: 4617-4622). Furthermore, as disclosed in WO00/18935, a promoter can be made stronger by introducing nucleotide substitutions for several nucleotides in a promoter region of a target gene so as to make the sequence closer to a consensus sequence. For example, the -35 region may be replaced with TTGACA or TTGCCA sequence, and the -10 region may be replaced with TATAAT and TATAAC sequence. In addition, it is known that translation efficiency of mRNA is significantly affected by substitution of several nucleotides in a sequence of a spacer between the ribosome binding site (RBS) and the translation initiation codon, in particular, a sequence immediately upstream of the initiation codon, and such a sequence may be modified.

Expression of a gene can also be enhanced by extending survival time of mRNA or by preventing degradation of an enzyme protein in cells. An expression control sequence such as a promoter upstream of the *sucE1* or *mdh* gene can also be identified by using a promoter search vector or gene analysis software such as GENETYX. Expressions of the *sucE1* gene and the *mdh* gene are enhanced by such promoter substitution or modification. Substitution for an expression control sequence can be performed by using, for example, a temperature-sensitive plasmid. Examples of the temperature-sensitive plasmid for coryneform bacteria include p48K, pSFKT2 (for these, Japanese Patent Laid-open No. 2000-262288), pHSC4 (French Patent Laid-open No. 1992-2667875 and Japanese Patent Laid-open No. 5-7491), and the like. These plasmids are autonomously replicable at least at 25°C, but are not autonomously replicable at 37°C, in coryneform bacteria. *Escherichia coli* AJ12571 harboring pHSC4 was deposited at the National Institute of Bioscience and Human-Technology, Agency of Industrial Science and Technology, Ministry of Trade and Industry (currently, International Patent Organism Depositary, National Institute of Advanced Industrial Science and Technology, Central 6, 1-1, Higashi 1-chome, Tsukuba-shi, Ibaraki-ken, Japan, 305-5466) on October 11, 1990 and assigned an accession number of FERM P-11763, and the deposit was then converted to an international deposit under the provisions of Budapest Treaty on August 26, 1991 and assigned an accession number of FERM BP-3524.

The modification of an expression control sequence may be combined with increase of copy numbers of the *sucE1* gene and *mdh* gene.

### Impartation of other properties

The bacterium used in the present invention may be modified so that the lactate dehydrogenase (LDH) activity is decreased, in addition to that expressions of sucE and *mdh* are enhanced. The expression "modified so that lactate dehydrogenase activity is decreased" used herein means that the LDH activity is lower than that of an LDH-unmodified strain such as a parent strain or a wild-type strain. The lactate dehydrogenase activity per cell is preferably decreased to 10% or lower as compared to that of an unmodified strain. The "decrease" includes complete deletion of the activity. Reduction of the LDH activity can be confirmed by measuring the LDH activity by a known method (Kanarek, L. and Hill, R.L., 1964, J. Biol. Chem., 239:4202). A coryneform bacterium in which LDH activity is decreased and expressions of the *sucE1* and *mdh* genes are enhanced can be obtained by, for example, transforming a bacterium of which LDH gene is disrupted with a recombinant vector containing the *sucE1* and *mdh* genes, as described in the examples mentioned later. However, either of the modification for reducing the LDH activity and the modification for enhancing expressions of the *sucE1* and *mdh* genes may be performed first.

In order to decrease the activity of LDH, a mutation that decreases the intracellular activity of LDH can be introduced into the LDH gene on a chromosome by a usual mutagenesis method. For example, it can be attained by deleting the gene coding for LDH on a chromosome, or modifying an expression control sequence such as a promoter and the Shine-Dalgarno (SD) sequence by gene recombination. Furthermore, it can also be attained by introducing a mutation for amino acid substitution (missense mutation), or a stop codon (nonsense mutation), or a frame shift mutation that adds or deletes one or two nucleotides into a region coding for LDH on a chromosome, or by deleting the gene partially or entirely (Journal of Biological Chemistry 272:8611-8617 (1997)). Furthermore, the LDH activity can also be decreased by constructing a gene coding for a mutant LDH of which coding region is deleted, and substituting the constructed gene for the normal LDH gene on a chromosome by homologous recombination or the like (Japanese Patent Laid-open No. 11-206385), or by introducing a transposon or IS factor into the genes, or by the method of using the *SacB* gene (Schafer, A. et al., Gene, 145 (1994) 69-73).

Gene substitution utilizing homologous recombination has already been established, and there are a method of using a linear DNA, a method of using a plasmid containing a temperature sensitive replication origin (U.S. Patent No. 6,303,383, Japanese Patent Laid-open No. 5-7491), and the like.

In addition, in a process for disrupting a gene as described above, levansucrase may be used as a marker for gene recombination (Schafer, A. et al., Gene, 145 (1994) 69-73).

The bacterium used in the present invention may be modified so that the pyruvate carboxylase (PC) activity is enhanced, in addition to that expressions of the *sucE1* and *mdh* genes are enhanced (Japanese Patent Laid-open No. 11-196888). The expression "modified so that pyruvate carboxylase activity is enhanced" means that the PC activity is higher than that of an unmodified strain such as a wild-type strain or a parent strain. The PC activity can be measured by, for example, a method of measuring decrease of NADH. A bacterium of which expressions of the *sucE1, mdh* and PC genes are enhanced can be produced by overexpressing the *sucE1, mdh* and PC genes in the bacterium in the same manner as that of the method described in Japanese Patent Laid-open No. 11-196888.

As the PC gene used in the method of the present invention, a gene having an already determined nucleotide sequence or a gene obtained by isolating a DNA fragment encoding a protein having the PC activity from a chromosome of a microorganism, animal, plant, or the like and determining the nucleotide sequence may be used. After the nucleotide sequence is determined, a gene synthesized on the basis of that sequence may also be used.

As the PC gene, for example, a PC gene derived from a coryneform bacterium such as *Corynebacterium glutamicum* (Peters-Wendisch, P.G. et al., 1998, Microbiology, vol. 144:915-927) (SEQ ID NO: 9) may be used. Furthermore, so long as functions of encoded PC, i.e., the properties of being involved in carbon dioxide fixation, are not substantially degraded, the PC gene may be a mutant or modified gene.

The PC genes obtained from any of bacteria other than *Corynebacterium glutamicum,* other microorganisms, animals and plants can also be used. In particular, sequences of the PC genes derived from microorganisms, animals and plants described below are known (references are indicated below), and they can be obtained by hybridization or amplification by PCR of the ORF regions.
Human [Biochem. Biophys. Res. Comm., 202, 1009-1014, (1999)]
Mouse [Proc. Natl. Acad. Sci. USA., 90, 1766-1779, (1993)]
Rat [GENE, 165, 331-332, (1995)]
Yeast: *Saccharomyces cerevisiae* [Mol. Gen. Genet., 229, 307-315, (1991)], *Schizosaccharomyces pombe* [DDBJ Accession No.; D78170]
*Bacillus stearothermophilus* [GENE, 191, 47-50, (1997)]
*Rhizobium etli* [J. Bacteriol., 178, 5960-5970, (1996)]

The PC gene can be enhanced in the same manner as those used for enhancing expressions of the *sucE1* and *mdh* genes described above.

The bacterium used in the present invention may be modified so that the acetyl-CoA hydrolase (ACH) activity is decreased, in addition to that expressions of *sucE1* and *mdh* are increased (WO2005/113744).

The "acetyl-CoA hydrolase (ACH) activity" means an activity for catalyzing the reaction to generate acetic acid from acetyl-CoA and water, and it is known that by-production of acetic acid is decreased by reducing the ACH activity. The expression "modified so that activity of acetyl-CoA hydrolase is decreased" means that the activity of acetyl-CoA hydrolase is lower than the specific activity of an unmodified strain such as a parent strain or a wild-type strain. The ACH activity per cell is preferably decreased to 50% or less, more preferably 30% or less, still more preferably 10% or less, as compared to that of an unmodified strain. The activity of acetyl-CoA hydrolase can be determined by reffering to the method of Gergely, J., et al. (Gergely, J., Hele, P. & Ramkrishnan, C.V. (1952) J. Biol. Chem. 198 p323-334). The term "decreased" includes complete deletion of the activity. The coryneform bacterium used in the present invention of which ACH activity is decreased, and expressions of the *sucE1* and *mdh* genes are enhanced can be obtained by producing a bacterium of which ACH gene is disrupted, and transforming the bacterium with a recombinant vector containing the *sucE1* and *mdh* genes. However, either the modification for reducing the ACH activity or the modification for enhancing expressions of the *sucE1* gene and the *mdh* gene may be performed first.

The bacterium used in the present invention may also be modified so that either or both of activities of phosphotransacetylase (henceforth referred to as PTA) and acetate kinase (henceforth referred to as ACK) are decreased in addition to that expressions of the *sucE1* gene and the *mdh* gene are enhanced. The phosphotransacetylase (PTA) activity means an activity for catalyzing the reaction to generate acetyl phosphate by transferring phosphate to acetyl-CoA (EC:2.3.1.8), and the acetate kinase (ACK) activity means an activity for catalyzing the reaction to generate acetic acid from acetyl phosphate and ADP (EC:2.7.2.1). The expression "phosphotransacetylase (PTA) activity is decreased" means that the PTA activity is lower than that of a PTA-unmodified strain. Although it is sufficient that the PTA activity per cell is decreased to be lower than that of a PTA-unmodified strain or a wild-type strain, it is preferably decreased to 50% or less, more preferably 10% or less, of the activity of a PTA-unmodified strain or a wild-type strain. The PTA activity may be completely deleted. The decrease of the PTA activity can be confirmed by measuring the PTA activity by the method of Klotzsch et al. (Klotzsch H.R., Meth. Enzymol., 12, 381-386 (1969)). Furthermore, the expression "acetate kinase (ACK) activity is decreased" means that the ACK activity is lower than that of an ACK-unmodified strain. Although it is sufficient that the ACK activity per cell is decreased to be lower than that of an ACK-unmodified strain or a wild-type strain, it is preferably decreased to 50% or less, more preferably 10% or less, as compared to that of an ACK-umnodified strain or a wild-type strain. The ACK activity may be completely deleted. The decrease in the ACK activity can be confirmed by measuring the ACK activity by the method of Irwin A. Rose (Rose, I.A., Meth. Enzymol., 1, 591-595 (1955)).

The coryneform bacterium used in the present invention in which either the activities of PTA and ACK was decreased, and expressions of the *sucE1* and *mdh* genes are enhanced can be obtained by, for example, producing a bacterium in which either the genes of PTA and ACK is disrupted, and transforming the bacterium with a recombinant vector containing the *sucE1* and *mdh* genes. However, either the modification for reducing either the PTA or ACK activity or the modification for enhancing expressions of the *sucE1* gene and the *mdh* gene may be performed first.

The bacterium used in the present invention may be modified so that the pyruvate oxidase (POX) activity is decreased, in addition to that expressions of *sucE1* and *mdh* are increased (WO2005/113745).

The "pyruvate oxidase activity" means an activity for catalyzing the reaction to generate acetic acid from pyruvic acid and water. The expression "modified so that activity of pyruvate oxidase hydrolase is decreased" means that the POX activity is lower than that of a POX-unmodified strain. The POX activity per cell is preferably decreased to 50% or less, more preferably 30% or less, particularly preferably 10% or less, as compared to that of an unmodified strain. The term "decreased" includes complete deletion of the activity. Examples of coryneform bacteria serving as control for comparison of the activity include, for example, *Brevibacterium lactofermentum* ATCC 13869 as a wild-type strain, and the *Brevibacterium lactofermentum* Δldh strain as an unmodified strain. The POX activity can be confirmed by measuring the activity by the method of Chang et al. (Chang Y. and Cronan J.E. JR, J. Bacteriol., 151, 1279-1289 (1982)). The coryneform bacterium used in the present invention in which POX activity is decreased, and expressions of the *sucE1* and *mdh* genes are enhanced can be obtained by producing a bacterium of which POX gene is disrupted, and transforming the bacterium with a recombinant vector containing the *sucE1* and *mdh* genes. However, either the modification for decreasing the POX activity or the modification for enhancing expressions of the *sucE1* gene and the *mdh* gene may be performed first.

Furthermore,
the bacterium used in the present invention may be modified so that the activity of succinate dehydrogenase (SDH) is enhanced (Japanese Patent Laid-open No. 2005-095169), in addition to that expressions of *sucE1* and *mdh* are enhanced. The expression "activity of succinate dehydrogenase is enhanced" means that the activity of SDH is higher than that of an unmodified strain such as a wild-type strain or a parent strain. The activity of SDH can be measured by a method of measuring decrease of NADH. A coryneform bacterium of which expressions of *sucE1, mdh* and SDH genes are enhanced can be produced by overexpressing the *sucE1, mdh* and SDH genes in the bacterium in the same manner as that of the method described in Japanese Patent Laid-open No. 11-196888.

As the SDH gene used for the method of the present invention, a gene of which nucleotide sequence is already determined, or a gene obtained by isolating a DNA fragment coding for a protein having the SDH activity from a chromosome of a microorganism, animal or plant, and determining the nucleotide sequence thereof can be used. Moreover, after the nucleotide sequence is once determined, a gene synthesized on the basis of the determined sequence can also be used. The sequences of the *sdh* operon of *Corynebacterium glutamicum* (GenBank accession Nos. NCgl0359 (*sdhC*), NCgl0360 (*sdhA*), NCgl0361 (*sdhB*)), and the sdh operon of *Brevibacterium flavum* (Japanese Patent Laid-open No. 2005-095169, European Patent Laid-open No. 1672077) are disclosed. It is known that, in *Escherichia coli,* if the activity of succinate reductase is insufficient, fumaric acid accumulation increases under an anaerobic condition (Journal of Industrial Microbiology & Biotechnology (2002) 28, 325-332). The expression "activity of succinate dehydrogenase is decreased" means that the activity of SDH is lower than that of an unmodified strain such as a wild-type strain or a parent strain. The succinate dehydrogenase activity per cell is preferably decreased to 10% or less of that of an unmodified strain. The activity of SDH can be measured by the aforementioned method.

The bacterium used in the present invention may have one of the "other properties" described above, or arbitrary two or three or more of them.

### Method for producing succinic acid

In the method for producing succinic acid of the present invention, above-described bacterium which has an ability to produce succinic acid and has been modified so that expressions of *sucE1* gene and *mdh* gene are enhanced or a product obtained by processing the bacterium is allowed to act on an organic raw material in a reaction mixture containing carbonate ions, bicarbonate ions, or carbon dioxide gas to produce succinic acid, and succinic acid is collected.

In the first aspect by culturing the microorganism in a medium containing carbonate ions, bicarbonate ions, or carbon dioxide gas, and an organic raw material, proliferation of the microorganism and production of succinic acid are simultaneously attained. In this aspect the medium corresponds to the reaction mixture. Proliferation of the microorganism and production of succinic acid may be simultaneously attained, or there may be a culture period in which proliferation of the microorganism is mainly attained, and a culture period in which production of succinic acid is mainly attained.

In the second aspect by allowing cells proliferated by culture in a medium to coexist with a reaction mixture containing carbonate ions, bicarbonate ions, or carbon dioxide gas, and an organic raw material, and thereby allowing the cells to act on the organic raw material in the reaction mixture, succinic acid is produced. In this embodiment, a product obtained by processing cells of the bacterium can also be used. Examples of the product obtained by processing cells include, for example, immobilized cells obtained by immobilizing cells with acrylamide, carragheenan, or the like, disrupted product obtained by disrupting cells, centrifugation supernatant of the disrupted product, fraction obtained by partial purification of the supernatant by ammonium sulfate treatment or the like, and the like.

Although the bacteria used for the culture may be those obtained on a solid medium such as agar medium by slant culture, those cultured beforehand in a liquid medium (seed culture) are preferred.

When an aerobic bacterium or a facultative anaerobic bacterium is used, cells are preferably cultured under aerobic conditions first, and then used for the succinic acid production reaction. Furthermore, although microaerobic induction is required for imparting succinic acid-producing activity to cells when cells cultured under aerobic conditions are used in the conventional methods, it is not necessary in the present invention. However, microaerobic induction may be performed. When microaerobic induction is performed, it is preferably performed for a period of 15 hours or shorter, more preferably 10 hours or shorter, further preferably 4 hours or shorter, although the period may differ depending on the condition of bacterium and degrees of microaerobic condition.

As the medium used for the culture, a medium usually used for culture of microorganisms can be used. For example, a generally-used medium obtained by adding natural nutrients such as meat extract, yeast extract and peptone, to a composition comprising inorganic salts such as ammonium sulfate, potassium phosphate and magnesium sulfate can be used.

In the aforementioned first aspect the carbon source added to the medium also serves as the organic raw material for the production of succinic acid.

In the aforementioned second aspect the cells after the culture are collected by centrifugation, membrane separation, or the like, and used for the succinic acid production reaction.

The organic raw material used in the method of the present invention is not particularly limited so long as a carbon source which the microorganism of the present invention can assimilate to produce succinic acid is used. However, fermentable carbohydrates including carbohydrates such as galactose, lactose, glucose, fructose, glycerol, sucrose, saccharose, starch and cellulose, polyalcohols such as glycerin, mannitol, xylitol and ribitol, and the like are usually used. Among these, glucose, fructose and glycerol are preferred, and glucose is particularly preferred.

Furthermore, a saccharified starch solution, molasses, or the like containing the aforementioned fermentable carbohydrates may also be used. Those fermentable carbohydrates may be used independently or in combination. Although concentration of the aforementioned organic raw material is not particularly limited, it is more advantageous that the concentration is as high as possible within such a range that culture of the microorganism and production of succinic acid are not inhibited. In the aforementioned first aspect concentration of the organic raw material in the medium is generally in the range of 5 to 30% (w/v), preferably 10 to 20% (w/v). Furthermore, in the aforementioned second aspect the concentration of the organic raw material in the reaction mixture is generally in the range of 5 to 30% (w/v), preferably 10 to 20% (w/v). Furthermore, the organic raw material may be supplemented along with decrease of the organic raw material with progress of the culture or reaction.

The aforementioned reaction mixture containing carbonate ions, bicarbonate ions, or carbon dioxide gas and the organic raw material is not particularly limited, and it may be, for example, a medium for culturing bacteria, or it may be a buffer such as phosphate buffer. The reaction mixture is preferably an aqueous solution containing a nitrogen source, inorganic salts, and the like. The nitrogen source is not particularly limited so long as a nitrogen source which the microorganism of the present invention can assimilate to produce succinic acid is chosen, and specific examples include various organic or inorganic nitrogen compounds such as ammonium salts, nitrates, urea, soybean hydrolysate, casein degradation products, peptone, yeast extract, meat extract, and corn steep liquor. As the inorganic salts, various phosphates, sulfates, and metallic salts such as those of magnesium, potassium, manganese, iron, and zinc are used. If necessary, growth-promoting factors including vitamins such as biotin, pantothenic acid, inositol, and nicotinic acid, nucleotides, amino acids and the like are added. In order to suppress foaming at the time of the reaction, it is desirable to add an appropriate amount of a commercially available antifoam to the medium.

pH of the reaction mixture can be adjusted by adding sodium carbonate, sodium bicarbonate, potassium carbonate, potassium bicarbonate, magnesium carbonate, sodium hydroxide, calcium hydroxide, magnesium hydroxide, or the like. Since pH for the reaction is usually 5 to 10, preferably 6 to 9.5, pH of the reaction mixture is adjusted to be within the aforementioned range with an alkaline substance, carbonate, urea, or the like even during the reaction, if needed.

As the reaction mixture used in the present invention, water, buffer, medium or the like is used, but a medium is most preferred. The medium can be made to contain, for example, the aforementioned organic raw material, and carbonate ions, bicarbonate ions, or carbon dioxide gas, and the reaction can be performed under an anaerobic condition. The carbonate or bicarbonate ions are supplied from magnesium carbonate, sodium carbonate, sodium bicarbonate, potassium carbonate, or potassium bicarbonate, which can also be used as a neutralizing agent. However, if necessary, carbonate or bicarbonate ions may also be supplied from carbonic acid or bicarbonic acid or salts thereof or carbon dioxide gas. Specific examples of the salts of carbonic acid or bicarbonic acid include, for example, magnesium carbonate, ammonium carbonate, sodium carbonate, potassium carbonate, ammonium bicarbonate, sodium bicarbonate, potassium bicarbonate, and the like. Carbonate ions or bicarbonate ions may be added at a concentration of 0.001 to 5 M, preferably 0.1 to 3 M, more preferably 1 to 2 M. When carbon dioxide gas is contained, carbon dioxide gas is contained in an amount of 50 mg to 25 g, preferably 100 mg to 15 g, more preferably 150 mg to 10 g, per litter of the solution.

The optimal growth temperature of the bacterium used for the reaction is generally in the range of 25 to 35°C. The reaction temperature is generally in the range of 25 to 40°C, preferably in the range of 30 to 37°C. Amount of bacterial cells in the reaction mixture is, although it is not particularly limited, 1 to 700 g/L, preferably 10 to 500 g/L, more preferably 20 to 400 g/L. The reaction time is preferably 1 to 168 hours, more preferably 3 to 72 hours. The reaction may be performed batchwise or in a column.

Culture of bacteria is preferably performed under an aerobic condition. On the other hand, the succinic acid production reaction may be performed under aerobic conditions, microaerobic conditions or anaerobic conditions. For the reaction under microaerobic conditions or anaerobic conditions, a method of performing the reaction in a sealed reaction vessel without aeration, a method of performing the reaction with supplying an inert gas such as nitrogen gas to the reaction mixture, a method of performing the reaction with supplying an inert gas containing carbon dioxide gas to the reaction mixture, and the like can be used.

The succinic acid accumulated in the reaction mixture (culture medium) may be separated and purified from the reaction mixture in a conventional manner. Specifically, solids such as bacterial cells can be removed by centrifugation, filtration, or the like, then the resultant solution can be desalted with an ion exchange resin or the like, and succinic acid can be separated and purified from the solution by crystallization or column chromatography.

Furthermore, in the present invention,
after succinic acid is produced by the method of the present invention described above, a polymerization reaction can be carried out by using the obtained succinic acid as a raw material to produce a polymer containing succinic acid. In recent years, with increase of environmentally friendly industrial products, polymers prepared from raw materials of plant origin have been attracting attentions. Succinic acid produced in the present invention can be converted into polymers such as polyesters and polyamides and used (Japanese Patent Laid-open No. 4-189822). Specific examples of succinic acid-containing polymers include succinic acid polyesters obtainable by polymerizing a diol such as butanediol and ethylene glycol and succinic acid, succinic acid polyamides obtainable by polymerizing a diamine such as hexamethylenediamine and succinic acid, and the like. In addition, succinic acid and succinic acid-containing polymers obtained by the production method of the present invention, and compositions containing these can be used for food additives, pharmaceutical agents, cosmetics, and the like.

### Examples

Hereinafter, the present invention will be explained more specifically with reference to examples.

### Example 1: Construction of sucE1 gene and mdh gene-enhanced strain

### <Construction of a plasmid for enhancement of sucE1>

An *sucE1* gene fragment in which the native promoter was replaced with the thioredoxin reductase (TRR) promoter was obtained by crossover PCR using synthetic DNAs designed by referring to the nucleotide sequences around the TRR and the *sucE1* gene of the genome sequence of *Corynebacterium glutamicum* ATCC 13032 already opened to public (GenBank Database Accession No.NC_003450).

A *sucE1* fragment containing the native promoter was amplified by PCR using the chromosome of *Brevibacterium flavum* MJ-233 as the template and the primers shown in SEQ ID NOS: 17 and 18. For PCR, Pyrobest DNA Polymerase (Takara Bio Inc.) was used, and the target PCR product was obtained by performing incubation at 94° for 3 minutes once, and then repeating a cycle consisting of denaturation at 94°C for 30 seconds, annealing at 60°C for 30 seconds and extension at 72°C for 1 minute 30 times. The PCR product was treated with *Sse*8387I, and the product was inserted into pVK9 at the *Sse*8387I site to construct a plasmid pVK9sucE1 carrying *sucE1* containing the native promoter. By treating this plasmid with *Xba*I and *Bst*XI, a part of ORF of *sucE1* containing the native promoter can be excised.

Furthermore, a fragment (A) containing the N-terminal sequence of *sucE1* was amplified by PCR using the chromosome of *Brevibacterium flavum* MJ-233 as the template and the primers shown in SEQ ID NOS: 19 and 20. Separately, a thioredoxin reductase (TRR) promoter fragment (B) was amplified by PCR using the chromosome of *Brevibacterium flavum* MJ-233 as the template and the primers shown in SEQ ID NOS: 21 and 22. In the PCR of both cases, PrimeSTAR HS DNA Polymerase (TaKaRa) was used, incubation was performed once at 98° for 2 minutes, and then a cycle consisting of denaturation at 98°C for 10 seconds, annealing at 55°C for 5 seconds and extension at 72°C for 20 seconds was repeated 30 times to obtain the target PCR products (A) and (B). The sequences of SEQ ID NOS: 19 and 22 are complementary to each other.

Then, a fragment containing the N-terminal sequence of *sucE1* in which the native promoter was replaced with the TRR promoter was constructed by crossover PCR using the fragments (A) and (B) as the templates and the primers shown in SEQ ID NOS: 20 and 21. In the PCR, PrimeSTAR HS DNA Polymerase (Takara Bio Inc.) was used, incubation was performed once at 98° for 2 minutes, and then a cycle consisting of denaturation at 98°C for 10 seconds, annealing at 55°C for 5 seconds and extension at 72°C for 40 seconds was repeated 30 times to obtain the target PCR product. The produced PCR product was purified in a conventional manner, treated with *Xba*I and BstXI, and then inserted into pVK9sucE1 at the *Xba*I and BstXI sites to construct a *sucE1* amplification plasmid, pVK9:PTRR-sucE1, in which the native promoter was replaced with the TRR promoter.

pVK9 is a shuttle vector obtained by blunt-ending the *Ava*II site of pHSG299 (Takara Bio), and inserting a fragment, obtained by excising a region autonomously replicable in coryneform bacteria contained in pHK4 (Japanese Patent Laid-open No. 5-007491) with BamHI and *Kpn*I and blunt-ending the excised region, into the above pHSG299 at the blunt-ended *Ava*II site.

### <Construction of a plasmid for enhancement of mdh>

An *mdh* gene fragment in which the native promoter was replaced with the elongation factor Tu (ET-Tu) promoter was obtained by crossover PCR using synthetic DNAs designed by referring to nucleotide sequences around EF-Tu and the *mdh* gene of the genome sequence of *Corynebacterium glutamicum* ATCC 13032 already opened to public (GenBank Database Accession No. NC_003450) as primers.

Specifically, an mdh fragment containing the native promoter was amplified by PCR using the chromosome of *Brevibacterium flavum* MJ-233 as the template and the primers shown in SEQ ID NOS: 23 and 24. In the PCR, Pyrobest DNA Polymerase (Takara Bio Inc.) was used, incubation was performed once at 94° for 2 minutes, and then a cycle consisting of denaturation at 94°C for 30 seconds, annealing at 57°C for 30 seconds and extension at 72°C for 1 minute and 30 seconds was repeated 30 times to obtain the target PCR product. The PCR product was treated with *XbaI* and *Sse*8387I, and then inserted into pVK9 at the *Xba*I and *Sse*8387I sites to construct a plasmid pVK9mdh carrying *mdh* containing the native promoter. A part of ORF of mdh containing the native promoter can be excised from this plasmid by treating the plasmid with BamHI and *Mlu*I*.*

Furthermore, a fragment (C) containing an N-terminal sequence of *mdh* was amplified by PCR using the chromosome of *Brevibacterium flavum* MJ-233 as the template and the primers shown in SEQ ID NOS: 25 and 26. Separately, an EF-Tu promoter fragment (D) was amplified by PCR using the chromosome of *Brevibacterium flavum* MJ-233 as the template and the primers shown in SEQ ID NOS: 27 and 28. In both PCR, PrimeSTAR HS DNA Polymerase (Takara Bio Inc.) was used, incubation was performed once at 98° for 2 minutes, and then a cycle consisting of denaturation at 98°C for 10 seconds, annealing at 55°C for 5 seconds and extension at 72°C for 20 seconds was repeated 30 times to obtain the target PCR products (C) and (D). The sequences of SEQ ID NOS: 25 and 28 are complementary to each other.

Then, a fragment containing the N-terminal sequence of *mdh* in which the native promoter was replaced with the EF-Tu promoter was constructed by crossover PCR using the fragments (C) and (D) as the templates and the primers shown in SEQ ID NOS: 27 and 26. In the PCR, PrimeSTAR HS DNA Polymerase (Takara Bio Inc.) was used, incubation was performed once at 98° for 2 minutes, and then a cycle consisting of denaturation at 98°C for 10 seconds, annealing at 55°C for 5 seconds and extension at 72°C for 40 seconds was repeated 30 times to obtain the target PCR product. The produced PCR product was purified in a conventional manner, treated with *BamHI* and *MluI,* and then inserted into pVK9mdh at the *BamH*I and *Mlu*I sites to construct a *mdh* amplification plasmid, pVK9:PEFTu-mdh, in which the native promoter was replaced with the EF-Tu promoter.

### <Construction of plasmid for enhancement of sucE1 + mdh>

A plasmid carrying both *sucE1* and *mdh* was constructed as follows.

First, an *mdh* gene fragment in which the native promoter was replaced with the ET-Tu promoter was obtained by crossover PCR using synthetic DNAs designed by referring to nucleotide sequences around EF-Tu and the *mdh* gene of the genome sequence of *Corynebacterium glutamicum* ATCC 13032 already opened to public (GenBank Database Accession No. NC_003450) as primers.

A fragment (G) containing orf of *mdh* was amplified by PCR using the chromosome of *Brevibacterium flavum* MJ-233 as the template and the primers shown in SEQ ID NOS: 25 and 29. In the PCR, PrimeSTAR HS DNA Polymerase (Takara Bio Inc.) was used, incubation was performed once at 98° for 2 minutes, and then a cycle consisting of denaturation at 98°C for 10 seconds, annealing at 55°C for 5 seconds and extension at 72°C for 1 minute was repeated 30 times to obtain the target PCR product (G). Separately, an EF-Tu promoter fragment (H) was amplified by PCR using the chromosome of *Brevibacterium flavum* MJ-233 as the template and the primers shown in SEQ ID NOS: 30 and 28. In the PCR, PrimeSTAR HS DNA Polymerase (Takara Bio Inc.) was used, incubation was performed once at 98° for 2 minutes, and then a cycle consisting of denaturation at 98°C for 10 seconds, annealing at 55°C for 5 seconds and extension at 72°C for 20 seconds was repeated 30 times to obtain the target PCR product (H). The sequences of SEQ ID NOS: 25 and 27 are complementary to each other.

Then, a fragment containing ORF of *mdh* in which the native promoter was replaced with the EF-Tu promoter was constructed by crossover PCR using the fragments (G) and (H) as the templates and the primer shown in SEQ ID NOS: 29 and 30. In the PCR, PrimeSTAR HS DNA Polymerase (Takara Bio Inc.) was used, incubation was performed once at 98° for 2 minutes, and then a cycle consisting of denaturation at 98°C for 10 seconds, annealing at 55°C for 5 seconds and extension at 72°C for 1 minute and 20 seconds was repeated 30 times to obtain the target PCR product. The produced PCR product was purified in a conventional manner, treated with *Spe*I and *Sse*8387I, and then inserted into pVK9:PTRR-sucE1 at the *Spe*I and *Sse*8387I sites to construct a plasmid carrying both *sucE1* and mdh, pVK9:PTRR-sucE1+PEFTu-mdh.

A plasmid carrying both *sucE1* and *mdh* can be constructed in the same manner by using chromosomal DNA of a strain other than the MJ-233 strain such as the *Corynebacterium glutamicum* AJ110655 strain as the template.

### <Construction of ldh gene-disrupted strain>

### (A) Cloning of fragment for ldh gene disruption

A gene fragment of the *ldh* gene deficient in the orf was obtained by crossover PCR using synthetic DNAs designed by referring to the nucleotide sequence around NCgl2817 of the genome sequence of *Corynebacterium glutamicum* ATCC 13032 already opened to public (GenBank Database Accession No. NC_003450) as primers. PCR was performed using the chromosomal DNA of *Brevibacterium lactofermentum* 2256 strain (ATCC 13869) as the template and the synthetic DNAs of SEQ ID NOS: 31 and 32 as primers to obtain an amplification product of an N-terminal sequence of the *ldh* gene. Separately, to obtain an amplification product of a C-terminal sequence of the *ldh* gene, PCR was performed by using the chromosomal DNA of *Brevibacterium lactofermentum* 2256 strain as the template and the synthetic DNAs of SEQ ID NOS: 33 and 34 as primers. In the PCR, Pyrobest DNA Polymerase (Takara Bio Inc.) was used, and each target PCR product was obtained by performing incubation at 94° for 3 minutes once, and then repeating a cycle consisting of denaturation at 94°C for 30 seconds, annealing at 60°C for 30 seconds and extension at 72°C for 1 minute 30 times. The sequences of SEQ ID NOS: 32 and 33 are complementary to each other. Then, to obtain an *ldh* gene fragment of which internal sequence was deleted, the above gene products of the N-terminal and C-terminal regions of *ldh* were mixed in approximately equimolar amounts, and PCR was performed using the mixture as the template and synthetic DNAs of SEQ ID NOS: 35 and 36 as primers to obtain an amplification product in which of orf of *ldh* was deleted. In the PCR, Pyrobest DNA Polymerase (Takara Bio Inc.) was used, and the target PCR product was obtained by performing incubation at 94° for 3 minutes once, and then repeating a cycle consisting of denaturation at 94°C for 30 seconds, annealing at 58°C for 30 seconds and extension at 72°C for 2 minute 30 times. The produced PCR product was purified in a conventional manner, then digested with *BamHI,* and inserted into a vector pBS4S for gene disruption (WO2007/046389) at the *BamHI* site to obtain a plasmid for *ldh* gene disruption, pBS4SΔldh56. The construction of this plasmid is shown in Fig. 1.

### (B) Production of ldh-disrupted strain

The pBS4SΔldh5 obtained in (A) described above does not contain a region that enables autonomous replication in cell of coryneform bacteria. Therefore, when a coryneform bacterium is transformed with this plasmid, a strain in which the plasmid is integrated into a chromosome by homologous recombination appears as a transformant, although it occurs at an extremely low frequency. The *Brevibacterium lactofermentum* 2256 strain was transformed with a high concentration of the plasmid pBS4SΔldh5 by the electric pulse method, and the transformed cells were applied on the CM-Dex agar medium (5 g/L of glucose, 10 g/L of polypeptone, 10 g/L of yeast extract, 1 g/L of KH₂PO₄, 0.4 g/L of MgSO₄·7H₂O, 0.01 g/L of FeSO₄·7H₂O, 0.01 g/L of MnSO₄·7H₂O, 3 g/L of urea, 1.2 g/L of soybean hydrolysate, pH 7.5 (KOH) containing 1.5% of agar) containing 50 µg/ml of kanamycin, and cultured at 31.5°C for about 24 hours. The plasmid used for the transformation of MJ-233 was obtained by transforming a dam methylase-deficient strain, *Escherichia coli* SCS110 strain (Stratagene), with pBS4SΔldh56, and extracting the plasmid from this strain. In the strains of colonies that appeared, the kanamycin resistance gene and the *sacB* gene derived from the plasmid were inserted into the genome, as a result of homologous recombination between the *ldh* gene fragment on the plasmid and the *ldh* gene on the genome of *Brevibacterium flavum* MJ-233 strain.

Then, these single recombinants were cultured overnight at 31.5°C in the CM-Dex liquid medium not containing kanamycin, then appropriately diluted, applied to 10% sucrose-containing Dex-S10 medium (100 g/L of sucrose, 10 g/L of polypeptone, 10 g/L of yeast extract, 1 g/L of KH₂PO₄, 0.4 g/L of MgSO₄·7H₂O, 0.01 g/L of FeSO₄·7H₂O, 0.01 g/L of MnSO₄·4H₂O, 3 g/L of urea, 1.2 g/L of soybean hydrolysate, 10 µg/L of biotin, pH 7.5 (KOH) containing 1.5% of agar) not containing kanamycin, and cultured at 31.5°C for about 24 hours to obtain clones exhibiting resistance to sucrose. These strains were strains which no longer expressed normal *sacB* gene, and included strains in which pBS4SΔldh5 was eliminated by the second homologous recombination. Furhter, the strains undergone the second homologous recombination included a strain in which the *ldh* gene was replaced with the deletion type gene derived from pBS4SΔldh56 and a strain in which the *ldh* gene was reverted to the wild type gene. Whether the *ldh* gene is the mutant or the wild-type can be easily confirmed by extracting chromosomal DNA from cells obtained by culture on the Dex-S10 agar medium, and detecting the *ldh* gene in the chromosomal DNA by PCR. Among the obtained double recombinant strains, a strain which provided, in amplification using the primers for PCR amplification of *ldh* gene (SEQ ID NOS: 31 and 34), a PCR product smaller than the PCR product obtained by using the chromosomal DNA of the MJ-233 strain was determined to be an *ldh*-deficient strain, and used in the following experiments. The obtained *ldh*-deficient strain was designated as AJ110655.

### <Construction of sucE1-amplified strain, mdh-amplified strain and sucE1+mdh-amplified strain>

The *Corynebacterium glutamicum* AJ110655 strain was transformed with pVK9:PTRR-sucE1, pVK9:PEFTu-mdh, pVK9:PTRR-sucE1+PEFTu-mdh obtained above and pVK9 by the electric pulse method, applied to the CM-Dex agar medium containing 25 µg/ml of kanamycin, and cultured at 31.5°C for about 24 hours to obtain strains into which each of the plasmids were introduced. The colonies that appeared were purified, and plasmids were extracted in a conventional manner to confirm introduction of each target plasmid.

### Example 2: Succinic acid production with sucE1 and mdh-amplified strain

Using strains obtained by introducing each of pVK9, the plasmid for enhancement of only *sucE1* gene, pVK9:PTRR-sucE1, the plasmid for enhancement of only mdh gene, pVK9:PEFTu-mdh, and the plasmid for enhancement of both *sucE1* and *mdh* genes, pVK9:PTRR-sucE1+PEFTu-mdh, into the *Corynebacterium glutamicum* AJ110655 strain, culture for succinic acid production was performed as follows. Cells of each strain obtained by culturing the strain on a CM-Dex plate medium were inoculated into 20 ml of a seed medium (25 g/L of glucose, 1.4 g/L of (NH₄)₂SO₄, 0.5 g/L of KH₂PO₄, 0.5 g/L of K₂HPO₄, 0.5 g/L of MgSO₄·7H₂O, 4 g/L of urea, 0.02 g/L of FeSO₄·7H₂O, 0.02 g/L of MnSO₄·7H₂O, 200 µg/L of biotin, 200 µg/L of VBl·HCl, 1 g/L of yeast extract, 1 g/L of casamino acid, 25 mg/L of kanamycin), and cultured at 31.5°C in a Sakaguchi flask under an aerobic condition with shaking for about 5 hours (aerobic culture) or 16 hours (microaerobic induction culture).

Then, 700 µl of the seed medium was isolated, and immediately mixed with 700 µl of a main medium (200 g/L of glucose, 30 g/L of Na sulfite, filtrated and mixed with 143 g/L as the final concentration of magnesium carbonate subjected to hot air sterilization) contained in a micro tube (Eppendorf tube), and culture was performed at 31.5°C with shaking. The culture was terminated after 48 hours, and amount of produced succinic acid was analyzed by liquid chromatography. Two of Shim-pack SCR-102H (Shimadzu) connected in series were used as the column, and a sample was eluted at 40°C with 5 mM p-toluenesulfonic acid. The eluate was neutralized with 20 mM Bis-Tris aqueous solution containing 5 mM p-toluenesulfonic acid and 100 µM EDTA, and succinic acid was quantified by measuring electric conductivity with CDD-10AD (Shimadzu).

When the microaerobically cultured cells obtained under the condition where the microaerobic induction was performed, i.e., obtained with the seed culture of 16 hours, were used, the succinic acid accumulation amounts per cell were equivalent in all the transformants. On the other hand, when the aerobically cultured cells obtained under the condition where the microaerobic induction was not performed, i.e., obtained with the seed culture of 5 hours, were used, only the pVK9:PTRR-sucE1+PEFTu-mdh-introduced strain showed succinic acid production higher than those obtained with the cells obtained with microaerobic induction, whereas none of the pVK9-introduced strain, pVK9:PTRR-sucE1-introduced strain and pVK9:PEFTu-mdh-introduced strain showed succinic acid production during the main culture. The results are shown in Table 1.

By these results, it was demonstrated that an activity for producing succinic acid under conditions of not performing microaerobic induction can be imparted by enhancing both *sucE1* and *mdh,* and it is effective for fermentative production of succinic acid.

**Table 1**

| Strain | Succinic acid accumulation amount (g/L/O.D.620=1) | |
|---|---|---|
| | Aerobically cultured cells | Microaerobically cultured cells |
| AJ110655/pVK9 | 0.6 | 1.6 |
| AJ110655/pVK9:PTRR-sucE1 | 0.7 | 1.8 |
| AJ110655/pVK9:PEFTu-mdh | 0.5 | 1.9 |
| AJ110655/pVK9:PTRR-sucE1+PEFTu-mdh | 2.0 | 1.7 |

### Explanation of Sequence Listing

SEQ ID NO: 1: Primer for *sucE1* gene amplification
SEQ ID NO: 2: Primer for *sucE1* gene amplification
SEQ ID NO: 3: Nucleotide sequence of *sucE1* gene of *B. flavum* MJ-233
SEQ ID NO: 4: Amino acid sequence encoded by the *sucE1* gene mentioned above
SEQ ID NO: 5: *sucE1* gene of *C. glutamicum* ATCC 13032
SEQ ID NO: 6: Amino acid sequence encoded by the *sucE1* gene mentioned above
SEQ ID NO: 7: Nucleotide sequence of *sucE1* gene derived from C. *efficiens* YS314
SEQ ID NO: 8: Amino acid sequence encoded by the *sucE1* gene mentioned above
SEQ ID NO: 9: Nucleotide sequence of PC gene of *C. glutamicum*
SEQ ID NO: 10: Amino acid sequence encoded by the PC gene mentioned above
SEQ ID NO: 11: Nucleotide sequence of *sucE1* gene of *C. diphtheriae gravis* NCTC 13129
SEQ ID NO: 12: Amino acid sequence encoded by the *sucE1* gene mentioned above
SEQ ID NO: 13: Nucleotide sequence of *mdh* gene of *B*. *flavum* MJ-233
SEQ ID NO: 14: Amino acid sequence encoded by the *mdh* gene mentioned above
SEQ ID NO: 15: Primer for *mdh* gene amplification
SEQ ID NO: 16: Primer for *mdh* gene amplification
SEQ ID NO: 17: Primer for amplification of *sucE1* fragment containing native promoter
SEQ ID NO: 18: Primer for amplification of *sucE1* fragment containing native promoter
SEQ ID NO: 19: Primer for amplification of fragment (A) containing N-terminal sequence of *sucE1*
SEQ ID NO: 20: Primer for amplification of fragment (A) containing the N terminal sequence of *sucE1*
SEQ ID NO: 21: Primer for amplification of TRR promoter fragment (B)
SEQ ID NO: 22: Primer for amplification of TRR promoter fragment (B)
SEQ ID NO: 23: Primer for amplification of *mdh* fragment containing native promoter
SEQ ID NO: 24: Primer for amplification of *mdh* fragment containing native promoter
SEQ ID NO: 25: Primer for amplification of fragment (C) or (G) containing N-terminal sequence of *mdh*
SEQ ID NO: 26: Primer for amplification of fragment (C) containing N-terminal sequence of *mdh*
SEQ ID NO: 27: Primer for amplification of EF-Tu promoter fragment (D)
SEQ ID NO: 28: Primer for amplification of EF-Tu promoter fragment (D) or (H)
SEQ ID NO: 29: Primer for amplification of fragment (G) containing *mdh* gene
SEQ ID NO: 30: Primer for amplification of EF-Tu promoter fragment (H)
SEQ ID NO: 31: Primer for production of fragment for *ldh* gene disruption (N-terminal sequence)
SEQ ID NO: 32: Primer for production of fragment for *ldh* gene disruption (N-terminal sequence)
SEQ ID NO: 33: Primer for production of fragment for *ldh* gene disruption (C-terminal sequence)
SEQ ID NO: 34: Primer for production of fragment for *ldh* gene disruption (C-terminal sequence)
SEQ ID NO: 35: Primer for production of fragment for *ldh* gene disruption
SEQ ID NO: 36: Primer for production of fragment for *ldh* gene disruption
SEQ ID NO: 37: Nucleotide sequence of superoxide dismutase gene promoter of *C. glutamicum* ATCC 13032
SEQ ID NO: 38: Nucleotide sequence of phosphoglycerate mutase gene promoter of *C. glutamicum* ATCC 13032
SEQ ID NO: 39: Nucleotide sequence of GroES-GroEL operon promoter of *C. glutamicum* ATCC 13032
SEQ ID NO: 40: Nucleotide sequence of peroxiredoxin gene promoter of *C. glutamicum* ATCC 13032
SEQ ID NO: 41: Nucleotide sequence of butanediol dehydrogenase gene promoter of *C. glutamicum* ATCC 13032
SEQ ID NO: 42: Nucleotide sequence of glyceraldehyde 3-phosphate dehydrogenase gene promoter of *C. glutamicum* ATCC 13032
SEQ ID NO: 43: Nucleotide sequence of thioredoxin reductase gene promoter of *C. glutamicum* ATCC 13032
SEQ ID NO: 44: Nucleotide sequence of fructose bisphosphate aldolase gene promoter of *C. glutamicum* ATCC 13032
SEQ ID NO: 45: Nucleotide sequence of EF-Tu gene promoter of *B. flavum* MJ-233

### Industrial Applicability

According to the method of the present invention, succinic acid can be quickly and highly efficiently produced from an organic raw material by using aerobically cultured cells without need of microaerobic induction. The obtained succinic acid can be used for food additives, pharmaceuticals, cosmetics, and the like. Moreover, succinic acid-containing polymers can also be produced by performing a polymerization reaction using the obtained succinic acid as a raw material.

## Claims

1. A method for producing succinic acid, which comprises allowing an aerobically cultured bacterium or cells thereof, which have been immobilized or disrupted, or a supernatant of said disrupted cells, or a partially purified fraction of said supernatant, wherein the bacterium has an ability to produce succinic acid and has been modified so that expressions of *sucE1* gene encoding succinate exporter and *mdh* gene encoding malate dehydrogenase are enhanced to act on an organic raw material in a reaction mixture containing carbonate ions, bicarbonate ions, or carbon dioxide gas to produce the succinic acid, and collecting the succinic acid.

2. The method according to claim 1, wherein the bacterium has been modified so that expression amounts of the *sucE1* gene and the *mdh* gene under aerobic conditions are increased by 1.5 times or more, respectively, as compared to an unmodified strain.

3. The method according to claim 1 or 2, wherein the bacterium is a bacterium selected from the group consisting of coryneform bacteria, *Bacillus* bacteria, and *Rhizobium* bacteria.

4. The method according to any one of claims 1 to 3, wherein expressions of the *sucE1* gene and/or the mdh gene is enhanced by increasing copy number of the *sucE1* gene and/or the *mdh* gene, by modifying an expression control sequence of these genes, or by replacing a promoter of these genes with a stronger promoter.

5. The method according to claim 4, wherein the stronger promoter is a constitutive expression promoter.

6. The method according to claim 4 or 5, wherein the stronger promoter is a promoter of a gene selected from genes coding for elongation factor Tu, cochaperonin GroES-chaperonin GroEL, thioredoxin reductase, phosphoglycerate mutase, peroxiredoxin, glycerol-3-phosphate dehydrogenase, 2,3-butanediol dehydrogenase, fructose bisphosphate aldolase, and superoxide dismutase.

7. The method according to any one of claims 1 to 6, wherein the *sucE1* gene is a DNA defined in (a) or (b):
(a) a DNA comprising the nucleotide sequence of the nucleotide numbers 571 to 2187 of SEQ ID NO: 3,
(b) a DNA which hybridizes with a nucleotide sequence complementary to the nucleotide sequence of the nucleotide numbers 571 to 2187 of SEQ ID NO: 3 under stringent conditions, which improves succinic acid-producing ability of the bacterium when expression thereof is enhanced in the bacterium.

8. The method according to any one of claims 1 to 7, wherein the *mdh* gene is a DNA defined in (c) or (d):
(c) a DNA comprising the nucleotide sequence of the nucleotide numbers 301 to 1287 of SEQ ID NO: 13,
(d) a DNA which hybridizes with a nucleotide sequence complementary to the nucleotide sequence of the nucleotide numbers 301 to 1287 of SEQ ID NO: 13 under stringent conditions, and codes for a protein having malate dehydrogenase activity.

9. The method according to any one of claims 1 to 8, wherein the bacterium has been further modified so that lactate dehydrogenase activity is decreased to 10% or less of the activity of an unmodified strain.

10. The method according to any one of claims 1 to 9, wherein the bacterium has been further modified so that pyruvate carboxylase activity is enhanced.

11. A method for producing a succinic acid-containing polymer, which comprises the steps of:
producing succinic acid by the method according to any one of claims 1 to 10, and
polymerizing the obtained succinic acid.

12. The method according to any one of claims 1 to 10, wherein the bacterium has been further modified so that succinate dehydrogenase activity is enhanced.

## Patentansprüche

1. Verfahren zur Produktion von Bernsteinsäure, umfassend die Stufe, in der zuglassen wird, dass ein aerob kultiviertes Bakterium oder Zellen davon, die immobilisiert oder zerstört sind, oder ein Überstand der zerstörten Zellen oder eine teilweise gereinigte Fraktion des Überstandes, wobei das Bakterium Bernsteinsäure produzieren kann und so modifiziert worden ist, dass die Expression des für Succinatexporter kodierenden *SucE1*-Gens und des für Malatdehydrogenase kodierenden *mdh*-Gens erhöht ist, auf ein organisches Ausgangsmaterial in einem Reaktionsgemisch, welches Carbonationen, Dicarbonationen oder Kohlendioxidgas enthält, unter Bildung von Bernsteinsäure wirkt, und eine Stufe, in der Bernsteinsäure gewonnen wird.

2. Verfahren nach Anspruch 1, wobei das Bakterium so modifiziert worden ist, dass die Expressionsmengen des *SucE1-*Gens und des *mdh*-Gens unter aeroben Bedingungen jeweils um das 1,5-Fache oder mehr im Vergleich zu einem nicht modifizierten Stamm erhöht sind.

3. Verfahren nach Anspruch 1 oder 2, wobei das Bakterium ein Bakterium ist, das aus der Gruppe ausgewählt ist, die aus coryneformen Bakterien, *Bacillusbakterien* und *Rhizobiumbakterien* besteht.

4. Verfahren nach einem der Ansprüche 1 bis 3, wobei die Expressionen des *SucE1*-Gens und/oder des *mdh*-Gens dadurch verstärkt sind, dass die Kopienzahl des *SucE1*-Gens und/oder des *mdh*-Gens erhöht ist/sind, die Expressionskontrollsequenz dieser Gene modifiziert ist oder ein Promotor dieser Gene durch einen stärkeren Promotor ersetzt ist.

5. Verfahren nach Anspruch 4, wobei der stärkere Promotor ein konstitutiver Expressionspromotor ist.

6. Verfahren nach Anspruch 5 oder 6, wobei der stärkere Promotor ein Promotor eines Gens ist, das unter Genen ausgewählt ist, die für den Elongationsfaktor Tu, Cochaperonin GroES-Chaperonin GroEL, Thioredoxinreductase, Phosphoglyceratmutase, Peroxiredoxin, Glycerol-3-phosphatdegydrogenase, 2,3-Butandioldeydrogenase, Fructosebisphosphataldolase und Superoxiddismutase kodieren.

7. Verfahren nach einem der Ansprüche 1 bis 6, wobei das *SucE1-Gen* eine in (a) oder in (b) definierte DNA ist:
(a) eine DNA, welche die Nukleotidsequenz der Nukleotidnummern 571 bis 2187 der SEQ ID NR: 3 umfasst,
(b) eine DNA, die unter stringenten Bedingungen mit einer zu der Nukleotidsequenz der Nukleotidnummern 571 bis 2187 der SEQ ID NR: 3 komplementären Nukleotidsequenz hybridisiert und welche die Bernsteinsäureproduktionsfähigkeit des Bakteriums verbessert, wenn ihre Expression in dem Bakterium erhöht wird.

8. Verfahren nach einem der Ansprüche 1 bis 7, wobei das *mdh-*Gen eine in (c) oder (d) definierte DNA ist:
(c) eine DNA, welche die Nukleotidsequenz der Nukleotidnummern 301 bis 1287 der SEQ ID NR: 13 enthält,
(d) eine DNA, die unter stringenten Bedingungen mit einer zu der Nukleotidsequenz der Nukleotidnummern 301 bis 1287 der SEQ ID NR: 13 komplementären Nukleotidsequenz hybridisiert und für ein Protein mit Malatdehydrogenaseaktivität kodiert.

9. Verfahren nach einem der Ansprüche 1 bis 8, wobei das Bakterium außerdem so modifiziert ist, dass die Lactatdehydrogenaseaktivität auf 10% oder weniger der Aktivität eines nicht modifizierten Stamms verringert ist.

10. Verfahren nach einem der Ansprüche 1 bis 9, wobei das Bakterium außerdem so modifiziert ist, dass die Pyruvatcarboxylaseaktivität verstärkt ist.

11. Verfahren zum Herstellen eines Bernsteinsäure enthaltenden Polymers, welches die folgenden Stufen umfasst:
das Produzieren von Bernsteinsäure durch das Verfahren nach einem der Ansprüche 1 bis 10 und
das Polymerisieren der erhaltenen Bernsteinsäure.

12. Verfahren nach einem der Ansprüche 1 bis 10, wobei das Bakterium außerdem so modifiziert ist, dass die Succinatdehydrogenaseaktivität verstärkt ist.

## Revendications

1. Procédé de production d'acide succinique, qui comprend l'étape consistant à permettre à une bactérie cultivée dans des conditions aérobies ou à des cellules de celle-ci, qui ont été immobilisées ou dissociées, ou à un surnageant desdites cellules dissociées, ou à une fraction partiellement purifiée dudit surnageant, dans lequel la bactérie a une capacité de produire de l'acide succinique et a été modifiée de manière à ce que l'expression du gène *sucE1* codant pour le succinate exportateur et du gène *mdh* codant pour la malate déshydrogénase soit améliorée pour agir sur une matière première organique dans un mélange réactionnel contenant des ions carbonate, des ions bicarbonate ou du dioxyde de carbone gazeux pour produire l'acide succinique, et le recueil de l'acide succinique.

2. Procédé selon la revendication 1, dans lequel la bactérie a été modifiée de manière à ce que les quantités d'expression du gène *sucE1* et du gène *mdh* dans des conditions aérobies soient augmentées de 1,5 fois ou plus, respectivement, en comparaison avec une souche non modifiée.

3. Procédé selon la revendication 1 ou 2, dans lequel la bactérie est une bactérie choisie dans le groupe constitué des bactéries corynéformes, des bactéries *Bacillus* et des bactéries *Rhizobium.*

4. Procédé selon l'une quelconque des revendications 1 à 3, dans lequel l'expression du gène *sucE1* et/ou du gène *mdh* est améliorée en augmentant le nombre de copies du gène *sucE1* et/ou du gène *mdh,* en modifiant une séquence de régulation de l'expression de ces gènes, ou en remplaçant un promoteur de ces gènes par un promoteur plus fort.

5. Procédé selon la revendication 4, dans lequel le promoteur le plus fort est un promoteur d'expression constitutif.

6. Procédé selon la revendication 4 ou 5, dans lequel le promoteur le plus fort est un promoteur d'un gène choisi parmi les gènes codant pour le facteur d'élongation Tu, la cochapéronine GroES-chapéronine GroEL, la thiorédoxine réductase, la phosphoglycérate mutase, la peroxyrédoxine, la glycérol-3-phosphate déshydrogénase, la 2,3-butanediol déshydrogénase, la fructose bisphosphate aldolase et la superoxyde dismutase.

7. Procédé selon l'une quelconque des revendications 1 à 6, dans lequel le gène *sucE1* est un ADN défini dans (a) ou (b) :
(a) un ADN comprenant la séquence nucléotidique des nucléotides numéros 571 à 2187 de la SEQ ID N° : 3,
(b) un ADN qui s'hybride avec une séquence nucléotidique complémentaire à la séquence nucléotidique des nucléotides numéros 571 à 2187 de la SEQ ID N° : 3 dans des conditions strictes, ce qui améliore la capacité de production d'acide succinique de la bactérie lorsque l'expression de celui-ci est améliorée dans la bactérie.

8. Procédé selon l'une quelconque des revendications 1 à 7, dans lequel le gène *mdh* est un ADN défini dans (c) ou (d) :
(c) un ADN comprenant la séquence nucléotidique des nucléotides numéros 301 à 1287 de la SEQ ID N° : 13,
(d) un ADN qui s'hybride avec une séquence nucléotidique complémentaire à la séquence nucléotidique des nucléotides numéros 301 à 1287 de la SEQ ID N° : 13 dans des conditions strictes, et qui code pour une protéine ayant une activité malate déshydrogénase.

9. Procédé selon l'une quelconque des revendications 1 à 8, dans lequel la bactérie a en outre été modifiée de manière à ce que l'activité lactate déshydrogénase soit augmentée jusqu'à 10 % ou moins de l'activité d'une souche non modifiée.

10. Procédé selon l'une quelconque des revendications 1 à 9, dans lequel la bactérie a en outre été modifiée de manière à ce que l'activité pyruvate carboxylase soit améliorée.

11. Procédé de production d'un polymère contenant de l'acide succinique, qui comprend les étapes consistant à :
produire de l'acide succinique par le procédé selon l'une quelconque des revendications 1 à 10, et
polymériser l'acide succinique obtenu.

12. Procédé selon l'une quelconque des revendications 1 à 10, dans lequel la bactérie a en outre été modifiée de manière à ce que l'activité succinate déshydrogénase soit améliorée.


### SEQUENCE LISTING

<110> Ajinomoto Co., Inc.
<120> A method for producing an organic acid
<130> C915-C8023
<150> JP2007-102668
   <151> 2007-04-10
<160> 45
<170> PatentIn version 3.3
<210> 1
   <211> 33
   <212> DNA
   <213> Artificial
<220>
   <223> primer
<400> 1
   gggcctgcag gaccaagacc gctgttgcag tga 33
<210> 2
   <211> 31
   <212> DNA
   <213> Artificial
<220>
   <223> primer
<400> 2
   gggcctgcag ggtattcaca ccagccccaa t 31
<210> 3
   <211> 2330
   <212> DNA
   <213> Brevibacterium flavum
<220>
   <221> CDS
   <222> (571)..(2187)
<400> 3
   gaccaagacc gctgttgcag tgaccactca gatggatgca gtgcaccgcg tcattggtct     60
   gtgtggtgtt gtgctggtcg gcgagggctc ccctcaccgc ctgaagccaa tgcttgcgca    120
   gcaaaagaag cgcctgaacc gcgtggcacc tggtgttccg gtgtatgaaa tcatcacggg    180
   caacggcgaa ggccagaccc ctatcgcgaa gctgcagcgt gaactggtca agctgcctcg    240
   caactacaag aagaacgacg tcgctgccct ggccgctcgc attgaggcta tggacaatgt    300
   cggaaacgct cctggcggat ctttgcctaa gggtccattg ccaaagggcg caagcatgtc    360
   cggtatgaac cgccgtgctc gccgacaggc tgaacgcagg ggcgaggctt aaagcctttt    420
   cgctttcgcg tcggcgccga agttttttaa aaagccgcac ttcaccaact tggatggggt    480
   gcggtttttg cgtgctctgg cttagggaac gtggccagtt ccacatcaaa taacgcagaa    540
   gtagtactta atccatgaga tcatgaatgg gtg agc ttc ctt gta gaa aat caa      594
                                    Val Ser Phe Leu Val Glu Asn Gln
                                    1               5
   tta ctc gcg ttg gtt gtc atc atg acg gtc gga cta ttg ctc ggc cgc       642
   Leu Leu Ala Leu Val Val Ile Met Thr Val Gly Leu Leu Leu Gly Arg
       10                  15                  20
   atc aaa att ttc ggg ttc cgc ctc ggt gtg gcc gct gta ttg ttt gtc       690
   Ile Lys Ile Phe Gly Phe Arg Leu Gly Val Ala Ala Val Leu Phe Val
   25                  30                  35                  40
   ggt ctg gcg ctg tcc acc att gag ccg gat att tcc gtc ccg ccg ctc       738
   Gly Leu Ala Leu Ser Thr Ile Glu Pro Asp Ile Ser Val Pro Pro Leu
                   45                  50                  55
   att tat gtt gtt gga ctg tca ctt ttt gtc tac acc atc gga ctg gag       786
   Ile Tyr Val Val Gly Leu Ser Leu Phe Val Tyr Thr Ile Gly Leu Glu
               60                  65                  70
   gcc ggc ccc gga ttc ttc acc tcc atg aaa acc aca ggt ctg cgc aac       834
   Ala Gly Pro Gly Phe Phe Thr Ser Met Lys Thr Thr Gly Leu Arg Asn
           75                  80                  85
   aac gca ctg acc ttg ggc gcc atc atc gcc acc acg gca ctc gca tgg       882
   Asn Ala Leu Thr Leu Gly Ala Ile Ile Ala Thr Thr Ala Leu Ala Trp
       90                  95                  100
   gca ctc atc acc gtt ttc aac atc gat gcc gcc tcc ggt gcc ggt atg       930
   Ala Leu Ile Thr Val Phe Asn Ile Asp Ala Ala Ser Gly Ala Gly Met
   105                 110                 115                 120
   ctc acc ggc gcg ctc acc aat acc cca gcc atg gcc gcg gtt gtt gac       978
   Leu Thr Gly Ala Leu Thr Asn Thr Pro Ala Met Ala Ala Val Val Asp
                   125                 130                 135
   gca ctg cct tcg ctt atc gac gac acc ggc cag ctt cac atc atc gcc      1026
   Ala Leu Pro Ser Leu Ile Asp Asp Thr Gly Gln Leu His Ile Ile Ala
               140                 145                 150
   gag ttg ccc gtc gtc gca tat tcc ttg gca tac ccc ctc gga gtg ctc      1074
   Glu Leu Pro Val Val Ala Tyr Ser Leu Ala Tyr Pro Leu Gly Val Leu
           155                 160                 165
   atc gtt att ctc tcc atc gcg atc ttc agc tct gtg ttc aaa gtg gat      1122
   Ile Val Ile Leu Ser Ile Ala Ile Phe Ser Ser Val Phe Lys Val Asp
       170                 175                 180
   cac aat aaa gag gcc gaa gaa gca ggt gtc gcg gtc cag gaa ctc aaa      1170
   His Asn Lys Glu Ala Glu Glu Ala Gly Val Ala Val Gln Glu Leu Lys
   185                 190                 195                 200
   ggc cgc cgc atc cgc gtc acc gtc gct gat ctt cca gcc ctg gag aat      1218
   Gly Arg Arg Ile Arg Val Thr Val Ala Asp Leu Pro Ala Leu Glu Asn
                   205                 210                 215
   atc cca gag ctg ctc aat ctc cac gtc att gtg tcc cgc gtg gaa cga      1266
   Ile Pro Glu Leu Leu Asn Leu His Val Ile Val Ser Arg Val Glu Arg
               220                 225                 230
   gac ggt gag caa ttc ata ccg ctt tat ggc gaa cac gca cgc atc ggc      1314
   Asp Gly Glu Gln Phe Ile Pro Leu Tyr Gly Glu His Ala Arg Ile Gly
           235                 240                 245
   gat gtc tta aca gtg gtg ggt gcc gat gaa gaa ctc aac cgc gcg gaa      1362
   Asp Val Leu Thr Val Val Gly Ala Asp Glu Glu Leu Asn Arg Ala Glu
       250                 255                 260
   aaa gcc atc ggt gaa ctc atc gac ggc gac ccc tac agc gat gtg gaa      1410
   Lys Ala Ile Gly Glu Leu Ile Asp Gly Asp Pro Tyr Ser Asp Val Glu
   265                 270                 275                 280
   ctt gat tac cga cgc atc ttc gtc tca aac aca gaa gtc gtg ggc act      1458
   Leu Asp Tyr Arg Arg Ile Phe Val Ser Asn Thr Glu Val Val Gly Thr
                   285                 290                 295
   ccc cta tcc aag ctc caa cca cta ttt aaa gac atg ctg atc acc cgt      1506
   Pro Leu Ser Lys Leu Gln Pro Leu Phe Lys Asp Met Leu Ile Thr Arg
               300                 305                 310
   atc agg cgc ggc gac aca gat ttg gtg gcc tcc ccc gac atg act ttg      1554
   Ile Arg Arg Gly Asp Thr Asp Leu Val Ala Ser Pro Asp Met Thr Leu
           315                 320                 325
   cag cta ggt gat cgt gtc cgc gtt gtc gca cca aca gag aag ctc cgc      1602
   Gln Leu Gly Asp Arg Val Arg Val Val Ala Pro Thr Glu Lys Leu Arg
       330                 335                 340
   gaa gca acc cga cta ctg ggc gat tcc tac aaa aaa ctc tcc gat ttc      1650
   Glu Ala Thr Arg Leu Leu Gly Asp Ser Tyr Lys Lys Leu Ser Asp Phe
   345                 350                 355                 360
   aac ctg ctc ccc ctc gct gcc ggc ctc atg atc ggt gtg ctt gtc ggc      1698
   Asn Leu Leu Pro Leu Ala Ala Gly Leu Met Ile Gly Val Leu Val Gly
                   365                 370                 375
   atg gta gaa ttc cca cta cca ggt gga agc tcc ctg aaa ctg ggt aac      1746
   Met Val Glu Phe Pro Leu Pro Gly Gly Ser Ser Leu Lys Leu Gly Asn
               380                 385                 390
   gca ggt gga ccg cta gtt gtt gcg ctg ctg ctc ggc atg att aat cgc      1794
   Ala Gly Gly Pro Leu Val Val Ala Leu Leu Leu Gly Met Ile Asn Arg
           395                 400                 405
   aca ggc aag ttc gtc tgg caa atc ccc tac gga gca aac ctt gcc ctt      1842
   Thr Gly Lys Phe Val Trp Gln Ile Pro Tyr Gly Ala Asn Leu Ala Leu
       410                 415                 420
   cgc caa ctg ggc atc aca cta ttt ttg gct gcc atc ggt acc tca gcg      1890
   Arg Gln Leu Gly Ile Thr Leu Phe Leu Ala Ala Ile Gly Thr Ser Ala
   425                 430                 435                 440
   ggc gca gga ttt cga tca gcg atc agc gac ccc caa tca ctc acc atc      1938
   Gly Ala Gly Phe Arg Ser Ala Ile Ser Asp Pro Gln Ser Leu Thr Ile
                   445                 450                 455
   atc ggc ttc ggt gcg ctg ctc act ttg ttc atc tcc atc acg gtc ctg      1986
   Ile Gly Phe Gly Ala Leu Leu Thr Leu Phe Ile Ser Ile Thr Val Leu
               460                 465                 470
   ttc gtt ggc cac aaa ctg atg aaa atc ccc ttc ggt gaa acc gct ggc      2034
   Phe Val Gly His Lys Leu Met Lys Ile Pro Phe Gly Glu Thr Ala Gly
           475                 480                 485
   atc ctc gcc ggt acg caa acc cac cct gct gtg ctg agt tat gtg tca      2082
   Ile Leu Ala Gly Thr Gln Thr His Pro Ala Val Leu Ser Tyr Val Ser
       490                 495                 500
   gat gcc tcc cgc aac gag ctc cct gcc atg ggt tat acc tct gtg tat      2130
   Asp Ala Ser Arg Asn Glu Leu Pro Ala Met Gly Tyr Thr Ser Val Tyr
   505                 510                 515                 520
   ccg ctg gcg atg atc gca aag atc ctg gcc gcc caa acg ttg ttg ttc      2178
   Pro Leu Ala Met Ile Ala Lys Ile Leu Ala Ala Gln Thr Leu Leu Phe
                   525                 530                 535
   cta ctt atc tagcattgac cccttaagcg cagaaggcga tttaaggggt              2227
   Leu Leu Ile
   tgggttttcc cgatgactag ttggtccaga gcgtgtattt gaggccctta ggggcgattc    2287
   tggggctgat gtttttgtgt ccattggggc tggtgtgaat acc                      2330
<210> 4
   <211> 539
   <212> PRT
   <213> Brevibacterium flavum
<400> 4
   Met Ser Phe Leu Val Glu Asn Gln Leu Leu Ala Leu Val Val Ile Met
   1               5                   10                  15
   Thr Val Gly Leu Leu Leu Gly Arg Ile Lys Ile Phe Gly Phe Arg Leu
               20                  25                  30
   Gly Val Ala Ala Val Leu Phe Val Gly Leu Ala Leu Ser Thr Ile Glu
           35                  40                  45
   Pro Asp Ile Ser Val Pro Pro Leu Ile Tyr Val Val Gly Leu Ser Leu
       50                  55                  60
   Phe Val Tyr Thr Ile Gly Leu Glu Ala Gly Pro Gly Phe Phe Thr Ser
   65                  70                  75                  80
   Met Lys Thr Thr Gly Leu Arg Asn Asn Ala Leu Thr Leu Gly Ala Ile
                   85                  90                  95
   Ile Ala Thr Thr Ala Leu Ala Trp Ala Leu Ile Thr Val Phe Asn Ile
               100                 105                 110
   Asp Ala Ala Ser Gly Ala Gly Met Leu Thr Gly Ala Leu Thr Asn Thr
           115                 120                 125
   Pro Ala Met Ala Ala Val Val Asp Ala Leu Pro Ser Leu Ile Asp Asp
       130                 135                 140
   Thr Gly Gln Leu His Ile Ile Ala Glu Leu Pro Val Val Ala Tyr Ser
   145                 150                 155                 160
   Leu Ala Tyr Pro Leu Gly Val Leu Ile Val Ile Leu Ser Ile Ala Ile
                   165                 170                 175
   Phe Ser Ser Val Phe Lys Val Asp His Asn Lys Glu Ala Glu Glu Ala
               180                 185                 190
   Gly Val Ala Val Gln Glu Leu Lys Gly Arg Arg Ile Arg Val Thr Val
           195                 200                 205
   Ala Asp Leu Pro Ala Leu Glu Asn Ile Pro Glu Leu Leu Asn Leu His
       210                 215                 220
   Val Ile Val Ser Arg Val Glu Arg Asp Gly Glu Gln Phe Ile Pro Leu
   225                 230                 235                 240
   Tyr Gly Glu His Ala Arg Ile Gly Asp Val Leu Thr Val Val Gly Ala
                   245                 250                 255
   Asp Glu Glu Leu Asn Arg Ala Glu Lys Ala Ile Gly Glu Leu Ile Asp
               260                 265                 270
   Gly Asp Pro Tyr Ser Asp Val Glu Leu Asp Tyr Arg Arg Ile Phe Val
           275                 280                 285
   Ser Asn Thr Glu Val Val Gly Thr Pro Leu Ser Lys Leu Gln Pro Leu
       290                 295                 300
   Phe Lys Asp Met Leu Ile Thr Arg Ile Arg Arg Gly Asp Thr Asp Leu
   305                 310                 315                 320
   Val Ala Ser Pro Asp Met Thr Leu Gln Leu Gly Asp Arg Val Arg Val
                   325                 330                 335
   Val Ala Pro Thr Glu Lys Leu Arg Glu Ala Thr Arg Leu Leu Gly Asp
               340                 345                 350
   Ser Tyr Lys Lys Leu Ser Asp Phe Asn Leu Leu Pro Leu Ala Ala Gly
           355                 360                 365
   Leu Met Ile Gly Val Leu Val Gly Met Val Glu Phe Pro Leu Pro Gly
       370                 375                 380
   Gly Ser Ser Leu Lys Leu Gly Asn Ala Gly Gly Pro Leu Val Val Ala
   385                 390                 395                 400
   Leu Leu Leu Gly Met Ile Asn Arg Thr Gly Lys Phe Val Trp Gln Ile
                   405                 410                 415
   Pro Tyr Gly Ala Asn Leu Ala Leu Arg Gln Leu Gly Ile Thr Leu Phe
               420                 425                 430
   Leu Ala Ala Ile Gly Thr Ser Ala Gly Ala Gly Phe Arg Ser Ala Ile
           435                 440                 445
   Ser Asp Pro Gln Ser Leu Thr Ile Ile Gly Phe Gly Ala Leu Leu Thr
       450                 455                 460
   Leu Phe Ile Ser Ile Thr Val Leu Phe Val Gly His Lys Leu Met Lys
   465                 470                 475                 480
   Ile Pro Phe Gly Glu Thr Ala Gly Ile Leu Ala Gly Thr Gln Thr His
                   485                 490                 495
   Pro Ala Val Leu Ser Tyr Val Ser Asp Ala Ser Arg Asn Glu Leu Pro
               500                 505                 510
   Ala Met Gly Tyr Thr Ser Val Tyr Pro Leu Ala Met Ile Ala Lys Ile
           515                 520                 525
   Leu Ala Ala Gln Thr Leu Leu Phe Leu Leu Ile
       530                 535
<210> 5
   <211> 1620
   <212> DNA
   <213> Corynebacterium glutamicum
<220>
   <221> CDS
   <222> (1)..(1617)
<400> 5
   gtg agc ttc ctt gta gaa aat caa tta ctc gcg ttg gtt gtc atc atg        48
   Val Ser Phe Leu Val Glu Asn Gln Leu Leu Ala Leu Val Val Ile Met
   1               5                   10                  15
   acg gtc gga cta ttg ctc ggc cgc atc aaa att ttc ggg ttc cgt ctc        96
   Thr Val Gly Leu Leu Leu Gly Arg Ile Lys Ile Phe Gly Phe Arg Leu
               20                  25                  30
   ggc gtc gcc gct gta ctg ttt gta ggt cta gcg cta tcc acc att gag       144
   Gly Val Ala Ala Val Leu Phe Val Gly Leu Ala Leu Ser Thr Ile Glu
           35                  40                  45
   ccg gat att tcc gtc cca tcc ctc att tac gtg gtt gga ctg tcg ctt       192
   Pro Asp Ile Ser Val Pro Ser Leu Ile Tyr Val Val Gly Leu Ser Leu
       50                  55                  60
   ttt gtc tac acg atc ggt ctg gaa gcc ggc cct gga ttc ttc acc tcc       240
   Phe Val Tyr Thr Ile Gly Leu Glu Ala Gly Pro Gly Phe Phe Thr Ser
   65                  70                  75                  80
   atg aaa acc act ggt ctg cgc aac aac gca ctg acc ttg ggc gcc atc       288
   Met Lys Thr Thr Gly Leu Arg Asn Asn Ala Leu Thr Leu Gly Ala Ile
                   85                  90                  95
   atc gcc acc acg gca ctc gca tgg gca ctc atc aca gtt ttg aac atc       336
   Ile Ala Thr Thr Ala Leu Ala Trp Ala Leu Ile Thr Val Leu Asn Ile
               100                 105                 110
   gat gcc gcc tcc ggc gcc ggc atg ctc acc ggc gcg ctc acc aac acc       384
   Asp Ala Ala Ser Gly Ala Gly Met Leu Thr Gly Ala Leu Thr Asn Thr
           115                 120                 125
   cca gcc atg gcc gca gtt gtt gac gca ctt cct tcg ctt atc gac gac       432
   Pro Ala Met Ala Ala Val Val Asp Ala Leu Pro Ser Leu Ile Asp Asp
       130                 135                 140
   acc ggc cag ctt cac ctc atc gcc gag ctg ccc gtc gtc gca tat tcc       480
   Thr Gly Gln Leu His Leu Ile Ala Glu Leu Pro Val Val Ala Tyr Ser
   145                 150                 155                 160
   ttg gca tac ccc ctc ggt gtg ctc atc gtt att ctc tcc atc gcc atc       528
   Leu Ala Tyr Pro Leu Gly Val Leu Ile Val Ile Leu Ser Ile Ala Ile
                   165                 170                 175
   ttc agc tct gtg ttc aaa gtc gac cac aac aaa gaa gcc gaa gaa gcg       576
   Phe Ser Ser Val Phe Lys Val Asp His Asn Lys Glu Ala Glu Glu Ala
               180                 185                 190
   ggc gtt gcg gtc cag gaa ctc aaa ggc cgt cgc atc cgc gtc acc gtc       624
   Gly Val Ala Val Gln Glu Leu Lys Gly Arg Arg Ile Arg Val Thr Val
           195                 200                 205
   gct gat ctt cca gcc ctg gag aac atc cca gag ctg ctc aac ctc cac      672
   Ala Asp Leu Pro Ala Leu Glu Asn Ile Pro Glu Leu Leu Asn Leu His
       210                 215                 220
   gtc att gtg tcc cga gtg gaa cga gac ggt gag caa ttc atc ccg ctt      720
   Val Ile Val Ser Arg Val Glu Arg Asp Gly Glu Gln Phe Ile Pro Leu
   225                 230                 235                 240
   tat ggc gaa cac gca cgc atc ggc gat gtc tta aca gtg gtg ggt gcc      768
   Tyr Gly Glu His Ala Arg Ile Gly Asp Val Leu Thr Val Val Gly Ala
                   245                 250                 255
   gat gaa gaa ctc aac cgc gcg gaa aaa gcc atc ggt gaa ctc att gac      816
   Asp Glu Glu Leu Asn Arg Ala Glu Lys Ala Ile Gly Glu Leu Ile Asp
               260                 265                 270
   ggc gac ccc tac agc aat gtg gaa ctt gat tac cga cgc atc ttc gtc      864
   Gly Asp Pro Tyr Ser Asn Val Glu Leu Asp Tyr Arg Arg Ile Phe Val
           275                 280                 285
   tca aac aca gca gtc gtg ggc act ccc cta tcc aag ctc cag cca ctg      912
   Ser Asn Thr Ala Val Val Gly Thr Pro Leu Ser Lys Leu Gln Pro Leu
       290                 295                 300
   ttt aaa gac atg ctg atc acc cgc atc agg cgc ggc gac aca gat ttg      960
   Phe Lys Asp Met Leu Ile Thr Arg Ile Arg Arg Gly Asp Thr Asp Leu
   305                 310                 315                 320
   gtg gcc tcc tcc gac atg act ttg cag ctc ggt gac cgt gtc cgc gtt     1008
   Val Ala Ser Ser Asp Met Thr Leu Gln Leu Gly Asp Arg Val Arg Val
                   325                 330                 335
   gtc gca cca gca gaa aaa ctc cgc gaa gca acc caa ttg ctc ggc gat     1056
   Val Ala Pro Ala Glu Lys Leu Arg Glu Ala Thr Gln Leu Leu Gly Asp
               340                 345                 350
   tcc tac aag aaa ctc tcc gat ttc aac ctg ctc cca ctc gct gcc ggc     1104
   Ser Tyr Lys Lys Leu Ser Asp Phe Asn Leu Leu Pro Leu Ala Ala Gly
           355                 360                 365
   ctc atg atc ggt gtg ctt gtc ggc atg gtg gag ttc cca cta cca ggt     1152
   Leu Met Ile Gly Val Leu Val Gly Met Val Glu Phe Pro Leu Pro Gly
       370                 375                 380
   gga agc tcc ctg aaa ctg ggt aac gca ggt gga ccg cta gtt gtt gcg     1200
   Gly Ser Ser Leu Lys Leu Gly Asn Ala Gly Gly Pro Leu Val Val Ala
   385                 390                 395                 400
   ctg ctg ctc ggc atg atc aat cgc aca ggc aag ttc gtc tgg caa atc     1248
   Leu Leu Leu Gly Met Ile Asn Arg Thr Gly Lys Phe Val Trp Gln Ile
                   405                 410                 415
   ccc tac gga gca aac ctt gcc ctt cgc caa ctg ggc atc aca cta ttt     1296
   Pro Tyr Gly Ala Asn Leu Ala Leu Arg Gln Leu Gly Ile Thr Leu Phe
               420                 425                 430
   ttg gct gcc atc ggt acc tca gcg ggc gca gga ttt cga tca gcg atc     1344
   Leu Ala Ala Ile Gly Thr Ser Ala Gly Ala Gly Phe Arg Ser Ala Ile
           435                 440                 445
   agc gac ccc caa tca ctc acc atc atc ggc ttc ggt gcg ctg ctc act     1392
   Ser Asp Pro Gln Ser Leu Thr Ile Ile Gly Phe Gly Ala Leu Leu Thr
       450                 455                 460
   ttg ttc atc tcc atc acg gtg ctg ttc gtt ggc cac aaa ctg atg aaa      1440
   Leu Phe Ile Ser Ile Thr Val Leu Phe Val Gly His Lys Leu Met Lys
   465                 470                 475                 480
   atc ccc ttc ggt gaa acc gct ggc atc ctc gcc ggt acg caa acc cac      1488
   Ile Pro Phe Gly Glu Thr Ala Gly Ile Leu Ala Gly Thr Gln Thr His
                   485                 490                 495
   cct gct gtg ctg agt tat gtg tca gat gcc tcc cgc aac gag ctc cct      1536
   Pro Ala Val Leu Ser Tyr Val Ser Asp Ala Ser Arg Asn Glu Leu Pro
               500                 505                 510
   gcc atg ggt tat acc tct gtg tat ccg ctg gcg atg atc gca aag atc      1584
   Ala Met Gly Tyr Thr Ser Val Tyr Pro Leu Ala Met Ile Ala Lys Ile
           515                 520                 525
   ctg gcc gcc caa acg ttg ttg ttc cta ctt atc tag                      1620
   Leu Ala Ala Gln Thr Leu Leu Phe Leu Leu Ile
       530                 535
<210> 6
   <211> 539
   <212> PRT
   <213> Corynebacterium glutamicum
<400> 6
   Met Ser Phe Leu Val Glu Asn Gln Leu Leu Ala Leu Val Val Ile Met
   1               5                   10                  15
   Thr Val Gly Leu Leu Leu Gly Arg Ile Lys Ile Phe Gly Phe Arg Leu
               20                  25                  30
   Gly Val Ala Ala Val Leu Phe Val Gly Leu Ala Leu Ser Thr Ile Glu
           35                  40                  45
   Pro Asp Ile Ser Val Pro Ser Leu Ile Tyr Val Val Gly Leu Ser Leu
       50                  55                  60
   Phe Val Tyr Thr Ile Gly Leu Glu Ala Gly Pro Gly Phe Phe Thr Ser
   65                  70                  75                  80
   Met Lys Thr Thr Gly Leu Arg Asn Asn Ala Leu Thr Leu Gly Ala Ile
                   85                  90                  95
   Ile Ala Thr Thr Ala Leu Ala Trp Ala Leu Ile Thr Val Leu Asn Ile
               100                 105                 110
   Asp Ala Ala Ser Gly Ala Gly Met Leu Thr Gly Ala Leu Thr Asn Thr
           115                 120                 125
   Pro Ala Met Ala Ala Val Val Asp Ala Leu Pro Ser Leu Ile Asp Asp
       130                 135                 140
   Thr Gly Gln Leu His Leu Ile Ala Glu Leu Pro Val Val Ala Tyr Ser
   145                 150                 155                 160
   Leu Ala Tyr Pro Leu Gly Val Leu Ile Val Ile Leu Ser Ile Ala Ile
                   165                 170                 175
   Phe Ser Ser Val Phe Lys Val Asp His Asn Lys Glu Ala Glu Glu Ala
               180                 185                 190
   Gly Val Ala Val Gln Glu Leu Lys Gly Arg Arg Ile Arg Val Thr Val
           195                 200                 205
   Ala Asp Leu Pro Ala Leu Glu Asn Ile Pro Glu Leu Leu Asn Leu His
       210                 215                 220
   Val Ile Val Ser Arg Val Glu Arg Asp Gly Glu Gln Phe Ile Pro Leu
   225                 230                 235                 240
   Tyr Gly Glu His Ala Arg Ile Gly Asp Val Leu Thr Val Val Gly Ala
                   245                 250                 255
   Asp Glu Glu Leu Asn Arg Ala Glu Lys Ala Ile Gly Glu Leu Ile Asp
               260                 265                 270
   Gly Asp Pro Tyr Ser Asn Val Glu Leu Asp Tyr Arg Arg Ile Phe Val
           275                 280                 285
   Ser Asn Thr Ala Val Val Gly Thr Pro Leu Ser Lys Leu Gln Pro Leu
       290                 295                 300
   Phe Lys Asp Met Leu Ile Thr Arg Ile Arg Arg Gly Asp Thr Asp Leu
   305                 310                 315                 320
   Val Ala Ser Ser Asp Met Thr Leu Gln Leu Gly Asp Arg Val Arg Val
                   325                 330                 335
   Val Ala Pro Ala Glu Lys Leu Arg Glu Ala Thr Gln Leu Leu Gly Asp
               340                 345                 350
   Ser Tyr Lys Lys Leu Ser Asp Phe Asn Leu Leu Pro Leu Ala Ala Gly
           355                 360                 365
   Leu Met Ile Gly Val Leu Val Gly Met Val Glu Phe Pro Leu Pro Gly
       370                 375                 380
   Gly Ser Ser Leu Lys Leu Gly Asn Ala Gly Gly Pro Leu Val Val Ala
   385                 390                 395                 400
   Leu Leu Leu Gly Met Ile Asn Arg Thr Gly Lys Phe Val Trp Gln Ile
                   405                 410                 415
   Pro Tyr Gly Ala Asn Leu Ala Leu Arg Gln Leu Gly Ile Thr Leu Phe
               420                 425                 430
   Leu Ala Ala Ile Gly Thr Ser Ala Gly Ala Gly Phe Arg Ser Ala Ile
           435                 440                 445
   Ser Asp Pro Gln Ser Leu Thr Ile Ile Gly Phe Gly Ala Leu Leu Thr
       450                 455                 460
   Leu Phe Ile Ser Ile Thr Val Leu Phe Val Gly His Lys Leu Met Lys
   465                 470                 475                 480
   Ile Pro Phe Gly Glu Thr Ala Gly Ile Leu Ala Gly Thr Gln Thr His
                   485                 490                 495
   Pro Ala Val Leu Ser Tyr Val Ser Asp Ala Ser Arg Asn Glu Leu Pro
               500                 505                 510
   Ala Met Gly Tyr Thr Ser Val Tyr Pro Leu Ala Met Ile Ala Lys Ile
           515                 520                 525
   Leu Ala Ala Gln Thr Leu Leu Phe Leu Leu Ile
       530                 535
<210> 7
   <211> 1629
   <212> DNA
   <213> Corynebacterium efficiens
<220>
   <221> CDS
   <222> (1)..(1626)
<400> 7
   gtg agc att ttc gtg gaa aat cag ttg ttg gcc ctg gtg gcc atc atg 48
   Val Ser Ile Phe Val Glu Asn Gln Leu Leu Ala Leu Val Ala Ile Met
   1 5 10 15
   ggt atc ggt ctg ttg ctc ggc cgg atc agg ttc ttc ggg ttc cgg ctc 96
   Gly Ile Gly Leu Leu Leu Gly Arg Ile Arg Phe Phe Gly Phe Arg Leu
   20 25 30
   ggg gtg gcg gcg gtg ttg ttc gtg ggt ctg gcc ttc tcc acc atc gaa 144
   Gly Val Ala Ala Val Leu Phe Val Gly Leu Ala Phe Ser Thr Ile Glu
   35 40 45
   ccg gac atc acg gtg cct ccc ctg atc tac gtg gtg ggc ctg gcc ctg 192
   Pro Asp Ile Thr Val Pro Pro Leu Ile Tyr Val Val Gly Leu Ala Leu
   50 55 60
   ttt gtg tac acc atc ggg ctg gag gcc ggc cgt gac ttc ttc agg tcg 240
   Phe Val Tyr Thr Ile Gly Leu Glu Ala Gly Arg Asp Phe Phe Arg Ser
   65 70 75 80
   ctg cgg tcc acc ggc ctg cgt aac aac ggg ctg gcc ctc ggt gcc atc 288
   Leu Arg Ser Thr Gly Leu Arg Asn Asn Gly Leu Ala Leu Gly Ala Ile
   85 90 95
   atc gcc acc acc gcg atc gcc tgg gtg gtc atc aag gcg ctg ggc ctg 336
   Ile Ala Thr Thr Ala Ile Ala Trp Val Val Ile Lys Ala Leu Gly Leu
   100 105 110
   gca ccc gcg acg ggt gcc ggc atg ttg acc ggt gcg ctg acc aac acc 384
   Ala Pro Ala Thr Gly Ala Gly Met Leu Thr Gly Ala Leu Thr Asn Thr
   115 120 125
   ccg gcg atg gcg gcg gtg gtc gac gcc cta ccg gcg ctt atc gac gac 432
   Pro Ala Met Ala Ala Val Val Asp Ala Leu Pro Ala Leu Ile Asp Asp
   130 135 140
   aac ccc acc gac gca gca cgc atc ctg gag ctg cct gtg gtg gcc tat 480
   Asn Pro Thr Asp Ala Ala Arg Ile Leu Glu Leu Pro Val Val Ala Tyr
   145 150 155 160
   tcc ctc acc tac ccg ctc ggt gtc ctg gtg gtg atc ctc acg atc gcg 528
   Ser Leu Thr Tyr Pro Leu Gly Val Leu Val Val Ile Leu Thr Ile Ala
   165 170 175
   gtc tgc ggg ggc ctg ttc aag gtc aac cat gag aag gag gcg aag aat 576
   Val Cys Gly Gly Leu Phe Lys Val Asn His Glu Lys Glu Ala Lys Asn
   180 185 190
   gcc ggc gtg gcc gtt cag gag ctg acg gga cgc cgt gtc cgg gtc acc 624
   Ala Gly Val Ala Val Gln Glu Leu Thr Gly Arg Arg Val Arg Val Thr
   195 200 205
   cgg gat gat ctg ccc gcc atc agc aat atc ccg gaa ctg ctg gac ctg 672
   Arg Asp Asp Leu Pro Ala Ile Ser Asn Ile Pro Glu Leu Leu Asp Leu
   210 215 220
   gag gtc atc gtc tcc cgt gtg gag cgc cgg ggg cag cat gac cag ttc 720
   Glu Val Ile Val Ser Arg Val Glu Arg Arg Gly Gln His Asp Gln Phe
   225                 230                 235                 240
   atc ccc gag gag ggt gat cgc acc cgg ttg ggt gac att ctc acc gtg      768
   Ile Pro Glu Glu Gly Asp Arg Thr Arg Leu Gly Asp Ile Leu Thr Val
                   245                 250                 255
   gtg ggc tcc gat gat gag ctc gag cgc gcg gtg ggt ctg ctc ggc gaa      816
   Val Gly Ser Asp Asp Glu Leu Glu Arg Ala Val Gly Leu Leu Gly Glu
               260                 265                 270
   ttc gtc gac ggc cat cca tac agc gat atc gat ctg gat tac cgc agg      864
   Phe Val Asp Gly His Pro Tyr Ser Asp Ile Asp Leu Asp Tyr Arg Arg
           275                 280                 285
   atc ttc gtc tct gat gaa tcc atg gtc ggt gtg ccc ttg gcg aaa ctg      912
   Ile Phe Val Ser Asp Glu Ser Met Val Gly Val Pro Leu Ala Lys Leu
       290                 295                 300
   cgc aac cgt att ccc ggc atg ttg atc acc cgg atc agg cgg ggt gac      960
   Arg Asn Arg Ile Pro Gly Met Leu Ile Thr Arg Ile Arg Arg Gly Asp
   305                 310                 315                 320
   acc gac ctg att gcg cat ccg gat atg acc ctc cag ctg ggt gat ctg     1008
   Thr Asp Leu Ile Ala His Pro Asp Met Thr Leu Gln Leu Gly Asp Leu
                   325                 330                 335
   gtc cgc gtg gtt gcc ccc gcc gag cgg atc aag gag gcc acc cac atc     1056
   Val Arg Val Val Ala Pro Ala Glu Arg Ile Lys Glu Ala Thr His Ile
               340                 345                 350
   ttc ggt gac tcc tac aaa cgc ctc gct gat ttc aat ctg gtt ccc ctg     1104
   Phe Gly Asp Ser Tyr Lys Arg Leu Ala Asp Phe Asn Leu Val Pro Leu
           355                 360                 365
   gtg gtc ggt ctc tcc ctc ggg gtg ctg gtg ggc atg atg gaa ttc ccc     1152
   Val Val Gly Leu Ser Leu Gly Val Leu Val Gly Met Met Glu Phe Pro
       370                 375                 380
   ctg ccc ggt gga agc gcc ctg tcc ctg ggc aat gcc ggt ggg ccc ctg     1200
   Leu Pro Gly Gly Ser Ala Leu Ser Leu Gly Asn Ala Gly Gly Pro Leu
   385                 390                 395                 400
   ttg atc gcg ctg ctg ctg ggg gcg atg ggc cgc acc ggc aag gtg gtc     1248
   Leu Ile Ala Leu Leu Leu Gly Ala Met Gly Arg Thr Gly Lys Val Val
                   405                 410                 415
   tgg cag atc ccc tac agt gcc aac ctc gcc ctc cga cag ctg ggc atc     1296
   Trp Gln Ile Pro Tyr Ser Ala Asn Leu Ala Leu Arg Gln Leu Gly Ile
               420                 425                 430
   acc atg ttt ctg gcg gcc atc ggg acg acc gcg ggt gcc ggg ttc cgg     1344
   Thr Met Phe Leu Ala Ala Ile Gly Thr Thr Ala Gly Ala Gly Phe Arg
           435                 440                 445
   tcc gcg ctg agc gat ccg gcc tcc ctg ctg atc atc ggg gtg ggt gcc     1392
   Ser Ala Leu Ser Asp Pro Ala Ser Leu Leu Ile Ile Gly Val Gly Ala
       450                 455                 460
   ctg ctg acc ctg gtg atc tcc gtg ctg gtt ctt gtc atc ggg cac aag     1440
   Leu Leu Thr Leu Val Ile Ser Val Leu Val Leu Val Ile Gly His Lys
   465                 470                 475                 480
   gtc atg cgt atc ccc ttc ggc gag acc gcc ggc atc ctc gcc ggc acc     1488
   Val Met Arg Ile Pro Phe Gly Glu Thr Ala Gly Ile Leu Ala Gly Thr
                   485                 490                 495
   cag acc cat cct gcg gtg ttg agc tac ata tcg gag gcc tcg cgc aat      1536
   Gln Thr His Pro Ala Val Leu Ser Tyr Ile Ser Glu Ala Ser Arg Asn
               500                 505                 510
   gaa ctg ccg gcg atg ggt tac acc tcc gtc tat ccc ctc gcc atg gtg      1584
   Glu Leu Pro Ala Met Gly Tyr Thr Ser Val Tyr Pro Leu Ala Met Val
           515                 520                 525
   gcg aag atc atc gcc gca cag gtg ctg ttg ttc ctg ctg ata tag          1629
   Ala Lys Ile Ile Ala Ala Gln Val Leu Leu Phe Leu Leu Ile
       530                 535                 540
<210> 8
   <211> 542
   <212> PRT
   <213> Corynebacterium efficiens
<400> 8
   Met Ser Ile Phe Val Glu Asn Gln Leu Leu Ala Leu Val Ala Ile Met
   1               5                   10                  15
   Gly Ile Gly Leu Leu Leu Gly Arg Ile Arg Phe Phe Gly Phe Arg Leu
               20                  25                  30
   Gly Val Ala Ala Val Leu Phe Val Gly Leu Ala Phe Ser Thr Ile Glu
           35                  40                  45
   Pro Asp Ile Thr Val Pro Pro Leu Ile Tyr Val Val Gly Leu Ala Leu
       50                  55                  60
   Phe Val Tyr Thr Ile Gly Leu Glu Ala Gly Arg Asp Phe Phe Arg Ser
   65                  70                  75                  80
   Leu Arg Ser Thr Gly Leu Arg Asn Asn Gly Leu Ala Leu Gly Ala Ile
                   85                  90                  95
   Ile Ala Thr Thr Ala Ile Ala Trp Val Val Ile Lys Ala Leu Gly Leu
               100                 105                 110
   Ala Pro Ala Thr Gly Ala Gly Met Leu Thr Gly Ala Leu Thr Asn Thr
           115                 120                 125
   Pro Ala Met Ala Ala Val Val Asp Ala Leu Pro Ala Leu Ile Asp Asp
       130                 135                 140
   Asn Pro Thr Asp Ala Ala Arg Ile Leu Glu Leu Pro Val Val Ala Tyr
   145                 150                 155                 160
   Ser Leu Thr Tyr Pro Leu Gly Val Leu Val Val Ile Leu Thr Ile Ala
                   165                 170                 175
   Val Cys Gly Gly Leu Phe Lys Val Asn His Glu Lys Glu Ala Lys Asn
               180                 185                 190
   Ala Gly Val Ala Val Gln Glu Leu Thr Gly Arg Arg Val Arg Val Thr
           195                 200                 205
   Arg Asp Asp Leu Pro Ala Ile Ser Asn Ile Pro Glu Leu Leu Asp Leu
       210                 215                 220
   Glu Val Ile Val Ser Arg Val Glu Arg Arg Gly Gln His Asp Gln Phe
   225                 230                 235                 240
   Ile Pro Glu Glu Gly Asp Arg Thr Arg Leu Gly Asp Ile Leu Thr Val
                   245                     250                     255
   Val Gly Ser Asp Asp Glu Leu Glu Arg Ala Val Gly Leu Leu Gly Glu
               260                     265                     270
   Phe Val Asp Gly His Pro Tyr Ser Asp Ile Asp Leu Asp Tyr Arg Arg
           275                     280                     285
   Ile Phe Val Ser Asp Glu Ser Met Val Gly Val Pro Leu Ala Lys Leu
       290                     295                     300
   Arg Asn Arg Ile Pro Gly Met Leu Ile Thr Arg Ile Arg Arg Gly Asp
   305                     310                     315                     320
   Thr Asp Leu Ile Ala His Pro Asp Met Thr Leu Gln Leu Gly Asp Leu
                   325                     330                     335
   Val Arg Val Val Ala Pro Ala Glu Arg Ile Lys Glu Ala Thr His Ile
               340                     345                     350
   Phe Gly Asp Ser Tyr Lys Arg Leu Ala Asp Phe Asn Leu Val Pro Leu
           355                     360                     365
   Val Val Gly Leu Ser Leu Gly Val Leu Val Gly Met Met Glu Phe Pro
       370                     375                     380
   Leu Pro Gly Gly Ser Ala Leu Ser Leu Gly Asn Ala Gly Gly Pro Leu
   385                     390                     395                     400
   Leu Ile Ala Leu Leu Leu Gly Ala Met Gly Arg Thr Gly Lys Val Val
                   405                     410                     415
   Trp Gln Ile Pro Tyr Ser Ala Asn Leu Ala Leu Arg Gln Leu Gly Ile
               420                     425                     430
   Thr Met Phe Leu Ala Ala Ile Gly Thr Thr Ala Gly Ala Gly Phe Arg
           435                     440                     445
   Ser Ala Leu Ser Asp Pro Ala Ser Leu Leu Ile Ile Gly Val Gly Ala
       450                     455                     460
   Leu Leu Thr Leu Val Ile Ser Val Leu Val Leu Val Ile Gly His Lys
   465                     470                     475                     480
   Val Met Arg Ile Pro Phe Gly Glu Thr Ala Gly Ile Leu Ala Gly Thr
                   485                     490                     495
   Gln Thr His Pro Ala Val Leu Ser Tyr Ile Ser Glu Ala Ser Arg Asn
               500                     505                     510
   Glu Leu Pro Ala Met Gly Tyr Thr Ser Val Tyr Pro Leu Ala Met Val
           515                     520                     525
   Ala Lys Ile Ile Ala Ala Gln Val Leu Leu Phe Leu Leu Ile
       530                     535                     540
<210> 9
   <211> 3423
   <212> DNA
   <213> Corynebacterium glutamicum
<220>
   <221> CDS
   <222> (1)..(3420)
<400> 9
   atg tcg act cac aca tct tca acg ctt cca gca ttc aaa aag atc ttg       48
   Met Ser Thr His Thr Ser Ser Thr Leu Pro Ala Phe Lys Lys Ile Leu
   1               5                   10                  15
   gta gca aac cgc ggc gaa atc gcg gtc cgt gct ttc cgt gca gca ctc       96
   Val Ala Asn Arg Gly Glu Ile Ala Val Arg Ala Phe Arg Ala Ala Leu
               20                  25                  30
   gaa acc ggt gca gcc acg gta gct att tac ccc cgt gaa gat cgg gga      144
   Glu Thr Gly Ala Ala Thr Val Ala Ile Tyr Pro Arg Glu Asp Arg Gly
           35                  40                  45
   tca ttc cac cgc tct ttt gct tct gaa gct gtc cgc att ggt act gaa      192
   Ser Phe His Arg Ser Phe Ala Ser Glu Ala Val Arg Ile Gly Thr Glu
       50                  55                  60
   ggc tca cca gtc aag gcg tac ctg gac atc gat gaa att atc ggt gca      240
   Gly Ser Pro Val Lys Ala Tyr Leu Asp Ile Asp Glu Ile Ile Gly Ala
   65                  70                  75                  80
   gct aaa aaa gtt aaa gca gat gct att tac ccg gga tat ggc ttc ctg      288
   Ala Lys Lys Val Lys Ala Asp Ala Ile Tyr Pro Gly Tyr Gly Phe Leu
                   85                  90                  95
   tct gaa aat gcc cag ctt gcc cgc gag tgc gcg gaa aac ggc att act      336
   Ser Glu Asn Ala Gln Leu Ala Arg Glu Cys Ala Glu Asn Gly Ile Thr
               100                 105                 110
   ttt att ggc cca acc cca gag gtt ctt gat ctc acc ggt gat aag tct      384
   Phe Ile Gly Pro Thr Pro Glu Val Leu Asp Leu Thr Gly Asp Lys Ser
           115                 120                 125
   cgt gcg gta acc gcc gcg aag aag gct ggt ctg cca gtt ttg gcg gaa      432
   Arg Ala Val Thr Ala Ala Lys Lys Ala Gly Leu Pro Val Leu Ala Glu
       130                 135                 140
   tcc acc ccg agc aaa aac atc gat gac atc gtt aaa agc gct gaa ggc      480
   Ser Thr Pro Ser Lys Asn Ile Asp Asp Ile Val Lys Ser Ala Glu Gly
   145                 150                 155                 160
   cag act tac ccc atc ttt gta aag gca gtt gcc ggt ggt ggc gga cgc      528
   Gln Thr Tyr Pro Ile Phe Val Lys Ala Val Ala Gly Gly Gly Gly Arg
                   165                 170                 175
   ggt atg cgc ttt gtt tct tca cct gat gag ctt cgc aaa ttg gca aca      576
   Gly Met Arg Phe Val Ser Ser Pro Asp Glu Leu Arg Lys Leu Ala Thr
               180                 185                 190
   gaa gca tct cgt gaa gct gaa gcg gca ttc ggc gac ggt tcg gta tat      624
   Glu Ala Ser Arg Glu Ala Glu Ala Ala Phe Gly Asp Gly Ser Val Tyr
           195                 200                 205
   gtc gag cgt gct gtg att aac ccc cag cac att gaa gtg cag atc ctt      672
   Val Glu Arg Ala Val Ile Asn Pro Gln His Ile Glu Val Gln Ile Leu
       210                 215                 220
   ggc gat cgc act gga gaa gtt gta cac ctt tat gaa cgt gac tgc tca      720
   Gly Asp Arg Thr Gly Glu Val Val His Leu Tyr Glu Arg Asp Cys Ser
   225                 230                 235                 240
   ctg cag cgt cgt cac caa aaa gtt gtc gaa att gcg cca gca cag cat      768
   Leu Gln Arg Arg His Gln Lys Val Val Glu Ile Ala Pro Ala Gln His
                   245                 250                 255
   ttg gat cca gaa ctg cgt gat cgc att tgt gcg gat gca gta aag ttc      816
   Leu Asp Pro Glu Leu Arg Asp Arg Ile Cys Ala Asp Ala Val Lys Phe
               260                 265                 270
   tgc cgc tcc att ggt tac cag ggc gcg gga act gtg gaa ttc ttg gtc      864
   Cys Arg Ser Ile Gly Tyr Gln Gly Ala Gly Thr Val Glu Phe Leu Val
           275                 280                 285
   gat gaa aag ggc aac cac gtt ttc atc gaa atg aac cca cgt atc cag      912
   Asp Glu Lys Gly Asn His Val Phe Ile Glu Met Asn Pro Arg Ile Gln
       290                 295                 300
   gtt gag cac acc gtg act gaa gaa gtc acc gag gtg gac ctg gtg aag      960
   Val Glu His Thr Val Thr Glu Glu Val Thr Glu Val Asp Leu Val Lys
   305                 310                 315                 320
   gcg cag atg cgc ttg gct gct ggt gca acc ttg aag gaa ttg ggt ctg     1008
   Ala Gln Met Arg Leu Ala Ala Gly Ala Thr Leu Lys Glu Leu Gly Leu
                   325                 330                 335
   acc caa gat aag atc aag acc cac ggt gcg gca ctg cag tgc cgc atc     1056
   Thr Gln Asp Lys Ile Lys Thr His Gly Ala Ala Leu Gln Cys Arg Ile
               340                 345                 350
   acc acg gaa gat cca aac aac ggc ttc cgc cca gat acc gga act atc     1104
   Thr Thr Glu Asp Pro Asn Asn Gly Phe Arg Pro Asp Thr Gly Thr Ile
           355                 360                 365
   acc gcg tac cgc tca cca ggc gga gct ggc gtt cgt ctt gac ggt gca     1152
   Thr Ala Tyr Arg Ser Pro Gly Gly Ala Gly Val Arg Leu Asp Gly Ala
       370                 375                 380
   gct cag ctc ggt ggc gaa atc acc gca cac ttt gac tcc atg ctg gtg     1200
   Ala Gln Leu Gly Gly Glu Ile Thr Ala His Phe Asp Ser Met Leu Val
   385                 390                 395                 400
   aaa atg acc tgc cgt ggt tcc gat ttt gaa act gct gtt gct cgt gca     1248
   Lys Met Thr Cys Arg Gly Ser Asp Phe Glu Thr Ala Val Ala Arg Ala
                   405                 410                 415
   cag cgc gcg ttg gct gag ttc acc gtg tct ggt gtt gca acc aac att     1296
   Gln Arg Ala Leu Ala Glu Phe Thr Val Ser Gly Val Ala Thr Asn Ile
               420                 425                 430
   ggt ttc ttg cgt gcg ttg ctg cgt gaa gag gac ttt act tcc aag cgc     1344
   Gly Phe Leu Arg Ala Leu Leu Arg Glu Glu Asp Phe Thr Ser Lys Arg
           435                 440                 445
   atc gcc acc gga ttt atc ggc gat cac cca cac ctc ctt cag gct cca     1392
   Ile Ala Thr Gly Phe Ile Gly Asp His Pro His Leu Leu Gln Ala Pro
       450                 455                 460
   cct gcg gat gat gag cag gga cgc atc ctg gat tac ttg gca gat gtc     1440
   Pro Ala Asp Asp Glu Gln Gly Arg Ile Leu Asp Tyr Leu Ala Asp Val
   465                 470                 475                 480
   acc gtg aac aag cct cat ggt gtg cgt cca aag gat gtt gca gca cca     1488
   Thr Val Asn Lys Pro His Gly Val Arg Pro Lys Asp Val Ala Ala Pro
                   485                 490                 495
   atc gat aag ctg ccc aac atc aag gat ctg cca ctg cca cgc ggt tcc     1536
   Ile Asp Lys Leu Pro Asn Ile Lys Asp Leu Pro Leu Pro Arg Gly Ser
               500                 505                 510
   cgt gac cgc ctg aag cag ctt gga cca gca gcg ttt gcc cgc gat ctc     1584
   Arg Asp Arg Leu Lys Gln Leu Gly Pro Ala Ala Phe Ala Arg Asp Leu
           515                 520                 525
   cgt gag cag gac gca ctg gca gtt act gat acc acc ttc cgc gat gca      1632
   Arg Glu Gln Asp Ala Leu Ala Val Thr Asp Thr Thr Phe Arg Asp Ala
       530                 535                 540
   cac cag tct ttg ctt gcg acc cga gtc cgc tca ttc gca ctg aag cct      1680
   His Gln Ser Leu Leu Ala Thr Arg Val Arg Ser Phe Ala Leu Lys Pro
   545                 550                 555                 560
   gcg gca gag gcc gtc gca aag ctg act cct gag ctt ttg tcc gtg gag      1728
   Ala Ala Glu Ala Val Ala Lys Leu Thr Pro Glu Leu Leu Ser Val Glu
                   565                 570                 575
   gcc tgg ggc ggt gcg acc tac gat gtg gcg atg cgt ttc ctc ttt gag      1776
   Ala Trp Gly Gly Ala Thr Tyr Asp Val Ala Met Arg Phe Leu Phe Glu
               580                 585                 590
   gat ccg tgg gac agg ctc gac gag ctg cgc gag gcg atg ccg aat gtg      1824
   Asp Pro Trp Asp Arg Leu Asp Glu Leu Arg Glu Ala Met Pro Asn Val
           595                 600                 605
   aac att cag atg ctg ctt cgc ggc cgc aac acc gtg gga tac acc cca      1872
   Asn Ile Gln Met Leu Leu Arg Gly Arg Asn Thr Val Gly Tyr Thr Pro
       610                 615                 620
   tac cca gac tcc gtc tgt cgc gcg ttt gtt aag gaa gct gcc acc tcc      1920
   Tyr Pro Asp Ser Val Cys Arg Ala Phe Val Lys Glu Ala Ala Thr Ser
   625                 630                 635                 640
   ggc gtg gac atc ttc cgc atc ttc gac gcg ctt aac gac gtc tcc cag      1968
   Gly Val Asp Ile Phe Arg Ile Phe Asp Ala Leu Asn Asp Val Ser Gln
                   645                 650                 655
   atg cgt cca gca atc gac gca gtc ctg gag acc aac acc gcg gtc gct      2016
   Met Arg Pro Ala Ile Asp Ala Val Leu Glu Thr Asn Thr Ala Val Ala
               660                 665                 670
   gaa gtg gct atg gct tat tct ggt gat ctt tcc gat ccg aat gaa aag      2064
   Glu Val Ala Met Ala Tyr Ser Gly Asp Leu Ser Asp Pro Asn Glu Lys
           675                 680                 685
   ctc tac acc ctg gat tac tac ctg aag atg gca gag gag atc gtc aag      2112
   Leu Tyr Thr Leu Asp Tyr Tyr Leu Lys Met Ala Glu Glu Ile Val Lys
       690                 695                 700
   tct ggc gct cac att ctg gct att aag gat atg gct ggt ctg ctt cgc      2160
   Ser Gly Ala His Ile Leu Ala Ile Lys Asp Met Ala Gly Leu Leu Arg
   705                 710                 715                 720
   cca gct gca gcc acc aag ctg gtc acc gca ctg cgc cgt gaa ttt gat      2208
   Pro Ala Ala Ala Thr Lys Leu Val Thr Ala Leu Arg Arg Glu Phe Asp
                   725                 730                 735
   ctg cca gtg cac gtg cac acc cac gac act gcg ggt ggc cag ctg gca      2256
   Leu Pro Val His Val His Thr His Asp Thr Ala Gly Gly Gln Leu Ala
               740                 745                 750
   acc tac ttt gct gca gct caa gct ggt gca gat gct gtt gac ggt gct      2304
   Thr Tyr Phe Ala Ala Ala Gln Ala Gly Ala Asp Ala Val Asp Gly Ala
           755                 760                 765
   tcc gca cca ctg tct ggc acc acc tcc cag cca tcc ctg tct gcc att      2352
   Ser Ala Pro Leu Ser Gly Thr Thr Ser Gln Pro Ser Leu Ser Ala Ile
       770                 775                 780
   gtt gct gca ttc gcg cac acc cgt cgc gat acc ggt ttg agc ctc gag      2400
   Val Ala Ala Phe Ala His Thr Arg Arg Asp Thr Gly Leu Ser Leu Glu
   785                 790                 795                 800
   gct gtt tct gac ctc gag cca tac tgg gaa gca gtg cgc gga ctg tac      2448
   Ala Val Ser Asp Leu Glu Pro Tyr Trp Glu Ala Val Arg Gly Leu Tyr
                   805                 810                 815
   ctg cca ttt gag tct gga acc cca ggc cca acc ggt cgc gtc tac cgc      2496
   Leu Pro Phe Glu Ser Gly Thr Pro Gly Pro Thr Gly Arg Val Tyr Arg
               820                 825                 830
   cac gaa atc cca ggc gga cag ctg tcc aac ctg cgt gca cag gcc acc      2544
   His Glu Ile Pro Gly Gly Gln Leu Ser Asn Leu Arg Ala Gln Ala Thr
           835                 840                 845
   gca ctg ggc ctt gcg gat cgt ttc gaa ctc atc gaa gac aac tac gcg      2592
   Ala Leu Gly Leu Ala Asp Arg Phe Glu Leu Ile Glu Asp Asn Tyr Ala
       850                 855                 860
   gca gtt aat gag atg ctg gga cgc cca acc aag gtc acc cca tcc tcc      2640
   Ala Val Asn Glu Met Leu Gly Arg Pro Thr Lys Val Thr Pro Ser Ser
   865                 870                 875                 880
   aag gtt gtt ggc gac ctc gca ctc cac ctc gtt ggt gcg ggt gtg gat      2688
   Lys Val Val Gly Asp Leu Ala Leu His Leu Val Gly Ala Gly Val Asp
                   885                 890                 895
   cca gca gac ttt gct gca gat cca caa aag tac gac atc cca gac tct      2736
   Pro Ala Asp Phe Ala Ala Asp Pro Gln Lys Tyr Asp Ile Pro Asp Ser
               900                 905                 910
   gtc atc gcg ttc ctg cgc ggc gag ctt ggt aac cct cca ggt ggc tgg      2784
   Val Ile Ala Phe Leu Arg Gly Glu Leu Gly Asn Pro Pro Gly Gly Trp
           915                 920                 925
   cca gag cca ctg cgc acc cgc gca ctg gaa ggc cgc tcc gaa ggc aag      2832
   Pro Glu Pro Leu Arg Thr Arg Ala Leu Glu Gly Arg Ser Glu Gly Lys
       930                 935                 940
   gca cct ctg acg gaa gtt cct gag gaa gag cag gcg cac ctc gac gct      2880
   Ala Pro Leu Thr Glu Val Pro Glu Glu Glu Gln Ala His Leu Asp Ala
   945                 950                 955                 960
   gat gat tcc aag gaa cgt cgc aac agc ctc aac cgc ctg ctg ttc ccg      2928
   Asp Asp Ser Lys Glu Arg Arg Asn Ser Leu Asn Arg Leu Leu Phe Pro
                   965                 970                 975
   aag cca acc gaa gag ttc ctc gag cac cgt cgc cgc ttc ggc aac acc      2976
   Lys Pro Thr Glu Glu Phe Leu Glu His Arg Arg Arg Phe Gly Asn Thr
               980                 985                 990
   tct gcg ctg gat gat cgt gaa ttc  ttc tac ggc ctg gtc  gaa ggc cgc    3024
   Ser Ala Leu Asp Asp Arg Glu Phe  Phe Tyr Gly Leu Val  Glu Gly Arg
           995                 1000                1005
   gag act  ttg atc cgc ctg cca  gat gtg cgc acc cca  ctg ctt gtt       3069
   Glu Thr  Leu Ile Arg Leu Pro  Asp Val Arg Thr Pro  Leu Leu Val
       1010                1015                1020
   cgc ctg  gat gcg atc tcc gag  cca gac gat aag ggt  atg cgc aat       3114
   Arg Leu  Asp Ala Ile Ser Glu  Pro Asp Asp Lys Gly  Met Arg Asn
       1025                1030                1035
   gtt gtg  gcc aac gtc aac ggc  cag atc cgc cca atg  cgt gtg cgt       3159
   Val Val  Ala Asn Val Asn Gly  Gln Ile Arg Pro Met  Arg Val Arg
       1040                 1045                 1050
   gac cgc  tcc gtt gag tct gtc  acc gca acc gca gaa  aag gca gat      3204
   Asp Arg  Ser Val Glu Ser Val  Thr Ala Thr Ala Glu  Lys Ala Asp
       1055                 1060                 1065
   tcc tcc  aac aag ggc cat gtt  gct gca cca ttc gct  ggt gtt gtc      3249
   Ser Ser  Asn Lys Gly His Val  Ala Ala Pro Phe Ala  Gly Val Val
       1070                 1075                 1080
   act gtg  act gtt gct gaa ggt  gat gag gtc aag gct  gga gat gca      3294
   Thr Val  Thr Val Ala Glu Gly  Asp Glu Val Lys Ala  Gly Asp Ala
       1085                 1090                 1095
   gtc gca  atc atc gag gct atg  aag atg gaa gca aca  atc act gct      3339
   Val Ala  Ile Ile Glu Ala Met  Lys Met Glu Ala Thr  Ile Thr Ala
       1100                 1105                 1110
   tct gtt  gac ggc aaa atc gat  cgc gtt gtg gtt cct  gct gca acg      3384
   Ser Val  Asp Gly Lys Ile Asp  Arg Val Val Val Pro  Ala Ala Thr
       1115                 1120                 1125
   aag gtg  gaa ggt ggc gac ttg  atc gtc gtc gtt tcc  taa              3423
   Lys Val  Glu Gly Gly Asp Leu  Ile Val Val Val Ser
       1130                 1135                 1140
<210> 10
   <211> 1140
   <212> PRT
   <213> Corynebacterium glutamicum
<400> 10
   Met Ser Thr His Thr Ser Ser Thr Leu Pro Ala Phe Lys Lys Ile Leu
   1               5                   10                  15
   Val Ala Asn Arg Gly Glu Ile Ala Val Arg Ala Phe Arg Ala Ala Leu
               20                  25                  30
   Glu Thr Gly Ala Ala Thr Val Ala Ile Tyr Pro Arg Glu Asp Arg Gly
           35                  40                  45
   Ser Phe His Arg Ser Phe Ala Ser Glu Ala Val Arg Ile Gly Thr Glu
       50                  55                  60
   Gly Ser Pro Val Lys Ala Tyr Leu Asp Ile Asp Glu Ile Ile Gly Ala
   65                  70                  75                  80
   Ala Lys Lys Val Lys Ala Asp Ala Ile Tyr Pro Gly Tyr Gly Phe Leu
                   85                  90                  95
   Ser Glu Asn Ala Gln Leu Ala Arg Glu Cys Ala Glu Asn Gly Ile Thr
               100                 105                 110
   Phe Ile Gly Pro Thr Pro Glu Val Leu Asp Leu Thr Gly Asp Lys Ser
           115                 120                 125
   Arg Ala Val Thr Ala Ala Lys Lys Ala Gly Leu Pro Val Leu Ala Glu
       130                 135                 140
   Ser Thr Pro Ser Lys Asn Ile Asp Asp Ile Val Lys Ser Ala Glu Gly
   145                 150                 155                 160
   Gln Thr Tyr Pro Ile Phe Val Lys Ala Val Ala Gly Gly Gly Gly Arg
   165 170 175
   Gly Met Arg Phe Val Ser Ser Pro Asp Glu Leu Arg Lys Leu Ala Thr
   180 185 190
   Glu Ala Ser Arg Glu Ala Glu Ala Ala Phe Gly Asp Gly Ser Val Tyr
   195 200 205
   Val Glu Arg Ala Val Ile Asn Pro Gln His Ile Glu Val Gln Ile Leu
   210 215 220
   Gly Asp Arg Thr Gly Glu Val Val His Leu Tyr Glu Arg Asp Cys Ser
   225 230 235 240
   Leu Gln Arg Arg His Gln Lys Val Val Glu Ile Ala Pro Ala Gln His
   245 250 255
   Leu Asp Pro Glu Leu Arg Asp Arg Ile Cys Ala Asp Ala Val Lys Phe
   260 265 270
   Cys Arg Ser Ile Gly Tyr Gln Gly Ala Gly Thr Val Glu Phe Leu Val
   275 280 285
   Asp Glu Lys Gly Asn His Val Phe Ile Glu Met Asn Pro Arg Ile Gln
   290 295 300
   Val Glu His Thr Val Thr Glu Glu Val Thr Glu Val Asp Leu Val Lys
   305 310 315 320
   Ala Gln Met Arg Leu Ala Ala Gly Ala Thr Leu Lys Glu Leu Gly Leu
   325 330 335
   Thr Gln Asp Lys Ile Lys Thr His Gly Ala Ala Leu Gln Cys Arg Ile
   340 345 350
   Thr Thr Glu Asp Pro Asn Asn Gly Phe Arg Pro Asp Thr Gly Thr Ile
   355 360 365
   Thr Ala Tyr Arg Ser Pro Gly Gly Ala Gly Val Arg Leu Asp Gly Ala
   370 375 380
   Ala Gln Leu Gly Gly Glu Ile Thr Ala His Phe Asp Ser Met Leu Val
   385 390 395 400
   Lys Met Thr Cys Arg Gly Ser Asp Phe Glu Thr Ala Val Ala Arg Ala
   405 410 415
   Gln Arg Ala Leu Ala Glu Phe Thr Val Ser Gly Val Ala Thr Asn Ile
   420 425 430
   Gly Phe Leu Arg Ala Leu Leu Arg Glu Glu Asp Phe Thr Ser Lys Arg
   435 440 445
   Ile Ala Thr Gly Phe Ile Gly Asp His Pro His Leu Leu Gln Ala Pro
   450 455 460
   Pro Ala Asp Asp Glu Gln Gly Arg Ile Leu Asp Tyr Leu Ala Asp Val
   465 470 475 480
   Thr Val Asn Lys Pro His Gly Val Arg Pro Lys Asp Val Ala Ala Pro
   485 490 495
   Ile Asp Lys Leu Pro Asn Ile Lys Asp Leu Pro Leu Pro Arg Gly Ser
   500 505 510
   Arg Asp Arg Leu Lys Gln Leu Gly Pro Ala Ala Phe Ala Arg Asp Leu
   515 520 525
   Arg Glu Gln Asp Ala Leu Ala Val Thr Asp Thr Thr Phe Arg Asp Ala
   530 535 540
   His Gln Ser Leu Leu Ala Thr Arg Val Arg Ser Phe Ala Leu Lys Pro
   545 550 555 560
   Ala Ala Glu Ala Val Ala Lys Leu Thr Pro Glu Leu Leu Ser Val Glu
                   565                 570                 575
   Ala Trp Gly Gly Ala Thr Tyr Asp Val Ala Met Arg Phe Leu Phe Glu
               580                 585                 590
   Asp Pro Trp Asp Arg Leu Asp Glu Leu Arg Glu Ala Met Pro Asn Val
           595                 600                 605
   Asn Ile Gln Met Leu Leu Arg Gly Arg Asn Thr Val Gly Tyr Thr Pro
       610                 615                 620
   Tyr Pro Asp Ser Val Cys Arg Ala Phe Val Lys Glu Ala Ala Thr Ser
   625                 630                 635                 640
   Gly Val Asp Ile Phe Arg Ile Phe Asp Ala Leu Asn Asp Val Ser Gln
                   645                 650                 655
   Met Arg Pro Ala Ile Asp Ala Val Leu Glu Thr Asn Thr Ala Val Ala
               660                 665                 670
   Glu Val Ala Met Ala Tyr Ser Gly Asp Leu Ser Asp Pro Asn Glu Lys
           675                 680                 685
   Leu Tyr Thr Leu Asp Tyr Tyr Leu Lys Met Ala Glu Glu Ile Val Lys
       690                 695                 700
   Ser Gly Ala His Ile Leu Ala Ile Lys Asp Met Ala Gly Leu Leu Arg
   705                 710                 715                 720
   Pro Ala Ala Ala Thr Lys Leu Val Thr Ala Leu Arg Arg Glu Phe Asp
                   725                 730                 735
   Leu Pro Val His Val His Thr His Asp Thr Ala Gly Gly Gln Leu Ala
               740                 745                 750
   Thr Tyr Phe Ala Ala Ala Gln Ala Gly Ala Asp Ala Val Asp Gly Ala
           755                 760                 765
   Ser Ala Pro Leu Ser Gly Thr Thr Ser Gln Pro Ser Leu Ser Ala Ile
       770                 775                 780
   Val Ala Ala Phe Ala His Thr Arg Arg Asp Thr Gly Leu Ser Leu Glu
   785                 790                 795                 800
   Ala Val Ser Asp Leu Glu Pro Tyr Trp Glu Ala Val Arg Gly Leu Tyr
                   805                 810                 815
   Leu Pro Phe Glu Ser Gly Thr Pro Gly Pro Thr Gly Arg Val Tyr Arg
               820                 825                 830
   His Glu Ile Pro Gly Gly Gln Leu Ser Asn Leu Arg Ala Gln Ala Thr
           835                 840                 845
   Ala Leu Gly Leu Ala Asp Arg Phe Glu Leu Ile Glu Asp Asn Tyr Ala
       850                 855                 860
   Ala Val Asn Glu Met Leu Gly Arg Pro Thr Lys Val Thr Pro Ser Ser
   865                 870                 875                 880
   Lys Val Val Gly Asp Leu Ala Leu His Leu Val Gly Ala Gly Val Asp
                   885                 890                 895
   Pro Ala Asp Phe Ala Ala Asp Pro Gln Lys Tyr Asp Ile Pro Asp Ser
               900                 905                 910
   Val Ile Ala Phe Leu Arg Gly Glu Leu Gly Asn Pro Pro Gly Gly Trp
           915                 920                 925
   Pro Glu Pro Leu Arg Thr Arg Ala Leu Glu Gly Arg Ser Glu Gly Lys
       930                 935                 940
   Ala Pro Leu Thr Glu Val Pro Glu Glu Glu Gln Ala His Leu Asp Ala
   945 950 955 960
   Asp Asp Ser Lys Glu Arg Arg Asn Ser Leu Asn Arg Leu Leu Phe Pro
   965 970 975
   Lys Pro Thr Glu Glu Phe Leu Glu His Arg Arg Arg Phe Gly Asn Thr
   980 985 990
   Ser Ala Leu Asp Asp Arg Glu Phe Phe Tyr Gly Leu Val Glu Gly Arg
   995 1000 1005
   Glu Thr Leu Ile Arg Leu Pro Asp Val Arg Thr Pro Leu Leu Val
   1010 1015 1020
   Arg Leu Asp Ala Ile Ser Glu Pro Asp Asp Lys Gly Met Arg Asn
   1025 1030 1035
   Val Val Ala Asn Val Asn Gly Gln Ile Arg Pro Met Arg Val Arg
   1040 1045 1050
   Asp Arg Ser Val Glu Ser Val Thr Ala Thr Ala Glu Lys Ala Asp
   1055 1060 1065
   Ser Ser Asn Lys Gly His Val Ala Ala Pro Phe Ala Gly Val Val
   1070 1075 1080
   Thr Val Thr Val Ala Glu Gly Asp Glu Val Lys Ala Gly Asp Ala
   1085 1090 1095
   Val Ala Ile Ile Glu Ala Met Lys Met Glu Ala Thr Ile Thr Ala
   1100 1105 1110
   Ser Val Asp Gly Lys Ile Asp Arg Val Val Val Pro Ala Ala Thr
   1115 1120 1125
   Lys Val Glu Gly Gly Asp Leu Ile Val Val Val Ser
   1130 1135 1140
<210> 11
   <211> 1626
   <212> DNA
   <213> Corynebacterium diphtheriae
<220>
   <221> CDS
   <222> (1)..(1626)
<400> 11
   gtg gat atc ttc gtg gca aac ccg ctg ttg gcg ctt ttt gtc atc atg 48
   Val Asp Ile Phe Val Ala Asn Pro Leu Leu Ala Leu Phe Val Ile Met
   1 5 10 15
   gcg gtt gga tta gcg atc ggt cag gtt aaa atc cga ggt ttt agt ctc 96
   Ala Val Gly Leu Ala Ile Gly Gln Val Lys Ile Arg Gly Phe Ser Leu
   20 25 30
   gga gtg gca gca gtg ctt ttt gca ggc gtt gga ttt gcg gct gta gag 144
   Gly Val Ala Ala Val Leu Phe Ala Gly Val Gly Phe Ala Ala Val Glu
   35 40 45
   cct gat att cac atc cca cac ttg gtg tac atc ttg gga ttg tct att 192
   Pro Asp Ile His Ile Pro His Leu Val Tyr Ile Leu Gly Leu Ser Ile
   50 55 60
   ttt gtg tac tcc atc ggt ttg gaa tca ggc cat gcg ttt ttc gcc ctg      240
   Phe Val Tyr Ser Ile Gly Leu Glu Ser Gly His Ala Phe Phe Ala Leu
   65                  70                  75                  80
   ttt aag tcg caa gga gtc aaa caa aac gcg ctc gct att acc gcg ttg      288
   Phe Lys Ser Gln Gly Val Lys Gln Asn Ala Leu Ala Ile Thr Ala Leu
                   85                  90                  95
   gcg ctg ata aca ggc att tct atc gct ttg ttt tcg ctg ata cat ctc      336
   Ala Leu Ile Thr Gly Ile Ser Ile Ala Leu Phe Ser Leu Ile His Leu
               100                 105                 110
   aac ggg gtc acc gct gct ggg ttg ttt aca ggc gcg gta aca aat aca      384
   Asn Gly Val Thr Ala Ala Gly Leu Phe Thr Gly Ala Val Thr Asn Thr
           115                 120                 125
   ccg gcc atg gct gcc gtg gtg gat tcg tta ccc agc atc ttt ggg gat      432
   Pro Ala Met Ala Ala Val Val Asp Ser Leu Pro Ser Ile Phe Gly Asp
       130                 135                 140
   gcg aac aag gta cac gag gtg gaa tcg ttg ccc tta gtt gca tat tcg      480
   Ala Asn Lys Val His Glu Val Glu Ser Leu Pro Leu Val Ala Tyr Ser
   145                 150                 155                 160
   ctg gcc tac ccc atc ggc gta ttg gga gtt atc gcg gca att ggg ttg      528
   Leu Ala Tyr Pro Ile Gly Val Leu Gly Val Ile Ala Ala Ile Gly Leu
                   165                 170                 175
   tgt gca aag tgg ttc cgt atc gat cac gtg caa gaa gct cac gac gca      576
   Cys Ala Lys Trp Phe Arg Ile Asp His Val Gln Glu Ala His Asp Ala
               180                 185                 190
   ggt gtt gct gtt gag gat ttg ttc acc cgt cag atc aag gtc aat cat      624
   Gly Val Ala Val Glu Asp Leu Phe Thr Arg Gln Ile Lys Val Asn His
           195                 200                 205
   gtg gtt acg ggc tct gat ctt gtg att gat atc cac cac acg ctg ggc      672
   Val Val Thr Gly Ser Asp Leu Val Ile Asp Ile His His Thr Leu Gly
       210                 215                 220
   cta gag att att gtc tcg cga att gag cgt gat ggt cag cag act ctc      720
   Leu Glu Ile Ile Val Ser Arg Ile Glu Arg Asp Gly Gln Gln Thr Leu
   225                 230                 235                 240
   cct act gcg tcg tct cgt atc cat atg gga gat gtg ttg tct gtg gtg      768
   Pro Thr Ala Ser Ser Arg Ile His Met Gly Asp Val Leu Ser Val Val
                   245                 250                 255
   ggc acg gcc gag gaa ctc gac aag gcc gcg cat gtg cta ggt gat ttg      816
   Gly Thr Ala Glu Glu Leu Asp Lys Ala Ala His Val Leu Gly Asp Leu
               260                 265                 270
   ctc cca ggc gat cct ttc cat ggt cac gat tta gat tat cgg cgc att      864
   Leu Pro Gly Asp Pro Phe His Gly His Asp Leu Asp Tyr Arg Arg Ile
           275                 280                 285
   ttt gtt tcc aat cag gat tta gtg ggc ata cct ctg gct aaa ctt cgg      912
   Phe Val Ser Asn Gln Asp Leu Val Gly Ile Pro Leu Ala Lys Leu Arg
       290                 295                 300
   cct cga ttg tca gga att ttg att act cgg gtc cgt cgt ggc gac cat      960
   Pro Arg Leu Ser Gly Ile Leu Ile Thr Arg Val Arg Arg Gly Asp His
   305                 310                 315                 320
   gac cat gtg gct act ccg gaa aca gtt ttg cag ctt ggc gac cgt gtt     1008
   Asp His Val Ala Thr Pro Glu Thr Val Leu Gln Leu Gly Asp Arg Val
                   325                     330                     335
   cgg gtg gtg gca gct cat gat cgt atg aaa tcc gtg acg gca ctg ttc      1056
   Arg Val Val Ala Ala His Asp Arg Met Lys Ser Val Thr Ala Leu Phe
               340                     345                     350
   ggc gat tcc tat cgt cgc ctt tct gat ttc aat ctt ttc cct ctc gtt      1104
   Gly Asp Ser Tyr Arg Arg Leu Ser Asp Phe Asn Leu Phe Pro Leu Val
           355                     360                     365
   gcc ggg ctt gcg ctt gga ttg ctc gtt ggc atg att gag gta cca ctt      1152
   Ala Gly Leu Ala Leu Gly Leu Leu Val Gly Met Ile Glu Val Pro Leu
       370                     375                     380
   cct ggc ggt gct gcg ttg tct tta gga agc gcc ggc ggc cca tta gta      1200
   Pro Gly Gly Ala Ala Leu Ser Leu Gly Ser Ala Gly Gly Pro Leu Val
   385                     390                     395                     400
   gta gct ctc gtt cta ggc gca gtg gga cgc tca ggt cgt ttt gtg tgg      1248
   Val Ala Leu Val Leu Gly Ala Val Gly Arg Ser Gly Arg Phe Val Trp
                   405                     410                     415
   cag gtt cct tac gga gca aac ttg gca ctg cga caa cta ggc atc acc      1296
   Gln Val Pro Tyr Gly Ala Asn Leu Ala Leu Arg Gln Leu Gly Ile Thr
               420                     425                     430
   ttg ttt ctc gcc gct att ggt acc act gca gga gct agt ttc cgt gca      1344
   Leu Phe Leu Ala Ala Ile Gly Thr Thr Ala Gly Ala Ser Phe Arg Ala
           435                     440                     445
   tcg cta tca gat ccc gca tcg ttg acc atc att gcc gtt ggt gcc atc      1392
   Ser Leu Ser Asp Pro Ala Ser Leu Thr Ile Ile Ala Val Gly Ala Ile
       450                     455                     460
   atc acc ttg aca ttg gct atc ttt gtt ctg gtt gtg ggc tat aag gtg      1440
   Ile Thr Leu Thr Leu Ala Ile Phe Val Leu Val Val Gly Tyr Lys Val
   465                     470                     475                     480
   atg aag atc ccg tac ggc caa aca gcg ggc atg ctt gcg ggt att caa      1488
   Met Lys Ile Pro Tyr Gly Gln Thr Ala Gly Met Leu Ala Gly Ile Gln
                   485                     490                     495
   acg cac cca gct gta ctg tcc tat gtt tct gcg atg acg aaa aat gat      1536
   Thr His Pro Ala Val Leu Ser Tyr Val Ser Ala Met Thr Lys Asn Asp
               500                     505                     510
   ctg ccg gca ttg ggc tac acc tcg gta tat cca ctg gcc atg atc gct      1584
   Leu Pro Ala Leu Gly Tyr Thr Ser Val Tyr Pro Leu Ala Met Ile Ala
           515                     520                     525
   aag atc att gca gca cag gtt gtg cta ttc gct ttg aca tag              1626
   Lys Ile Ile Ala Ala Gln Val Val Leu Phe Ala Leu Thr
       530                     535                     540
<210> 12
   <211> 541
   <212> PRT
   <213> Corynebacterium diphtheriae
<400> 12
   Met Asp Ile Phe Val Ala Asn Pro Leu Leu Ala Leu Phe Val Ile Met
   1               5                   10                  15
   Ala Val Gly Leu Ala Ile Gly Gln Val Lys Ile Arg Gly Phe Ser Leu
               20                  25                  30
   Gly Val Ala Ala Val Leu Phe Ala Gly Val Gly Phe Ala Ala Val Glu
           35                  40                  45
   Pro Asp Ile His Ile Pro His Leu Val Tyr Ile Leu Gly Leu Ser Ile
       50                  55                  60
   Phe Val Tyr Ser Ile Gly Leu Glu Ser Gly His Ala Phe Phe Ala Leu
   65                  70                  75                  80
   Phe Lys Ser Gln Gly Val Lys Gln Asn Ala Leu Ala Ile Thr Ala Leu
                   85                  90                  95
   Ala Leu Ile Thr Gly Ile Ser Ile Ala Leu Phe Ser Leu Ile His Leu
               100                 105                 110
   Asn Gly Val Thr Ala Ala Gly Leu Phe Thr Gly Ala Val Thr Asn Thr
           115                 120                 125
   Pro Ala Met Ala Ala Val Val Asp Ser Leu Pro Ser Ile Phe Gly Asp
       130                 135                 140
   Ala Asn Lys Val His Glu Val Glu Ser Leu Pro Leu Val Ala Tyr Ser
   145                 150                 155                 160
   Leu Ala Tyr Pro Ile Gly Val Leu Gly Val Ile Ala Ala Ile Gly Leu
                   165                 170                 175
   Cys Ala Lys Trp Phe Arg Ile Asp His Val Gln Glu Ala His Asp Ala
               180                 185                 190
   Gly Val Ala Val Glu Asp Leu Phe Thr Arg Gln Ile Lys Val Asn His
           195                 200                 205
   Val Val Thr Gly Ser Asp Leu Val Ile Asp Ile His His Thr Leu Gly
       210                 215                 220
   Leu Glu Ile Ile Val Ser Arg Ile Glu Arg Asp Gly Gln Gln Thr Leu
   225                 230                 235                 240
   Pro Thr Ala Ser Ser Arg Ile His Met Gly Asp Val Leu Ser Val Val
                   245                 250                 255
   Gly Thr Ala Glu Glu Leu Asp Lys Ala Ala His Val Leu Gly Asp Leu
               260                 265                 270
   Leu Pro Gly Asp Pro Phe His Gly His Asp Leu Asp Tyr Arg Arg Ile
           275                 280                 285
   Phe Val Ser Asn Gln Asp Leu Val Gly Ile Pro Leu Ala Lys Leu Arg
       290                 295                 300
   Pro Arg Leu Ser Gly Ile Leu Ile Thr Arg Val Arg Arg Gly Asp His
   305                 310                 315                 320
   Asp His Val Ala Thr Pro Glu Thr Val Leu Gln Leu Gly Asp Arg Val
                   325                 330                 335
   Arg Val Val Ala Ala His Asp Arg Met Lys Ser Val Thr Ala Leu Phe
               340                 345                 350
   Gly Asp Ser Tyr Arg Arg Leu Ser Asp Phe Asn Leu Phe Pro Leu Val
           355                 360                 365
   Ala Gly Leu Ala Leu Gly Leu Leu Val Gly Met Ile Glu Val Pro Leu
       370                 375                 380
   Pro Gly Gly Ala Ala Leu Ser Leu Gly Ser Ala Gly Gly Pro Leu Val
   385                 390                 395                 400
   Val Ala Leu Val Leu Gly Ala Val Gly Arg Ser Gly Arg Phe Val Trp
                   405                 410                 415
   Gln Val Pro Tyr Gly Ala Asn Leu Ala Leu Arg Gln Leu Gly Ile Thr
               420                 425                 430
   Leu Phe Leu Ala Ala Ile Gly Thr Thr Ala Gly Ala Ser Phe Arg Ala
           435                 440                 445
   Ser Leu Ser Asp Pro Ala Ser Leu Thr Ile Ile Ala Val Gly Ala Ile
       450                 455                 460
   Ile Thr Leu Thr Leu Ala Ile Phe Val Leu Val Val Gly Tyr Lys Val
   465                 470                 475                 480
   Met Lys Ile Pro Tyr Gly Gln Thr Ala Gly Met Leu Ala Gly Ile Gln
                   485                 490                 495
   Thr His Pro Ala Val Leu Ser Tyr Val Ser Ala Met Thr Lys Asn Asp
               500                 505                 510
   Leu Pro Ala Leu Gly Tyr Thr Ser Val Tyr Pro Leu Ala Met Ile Ala
           515                 520                 525
   Lys Ile Ile Ala Ala Gln Val Val Leu Phe Ala Leu Thr
       530                 535                 540
<210> 13
   <211> 1502
   <212> DNA
   <213> Brevibacterium flavum
<220>
   <221> CDS
   <222> (301)..(1287)
<400> 13
   cctttcgggt aacgagaaaa cgtgaactag aaacggagtc aaagtaaata tcaaaggtaa      60
   caccatcggt aaatccaaac tgacaactat aaatggtgcc cgatatcagg aaaaattgct     120
   tgcacaccgc gccgattccc catgatgccc taacatcttg caggtaaggg gtacatattg     180
   gggcaattcg ggggtgattt tgcagtatcg tcaagatcac ccaaaactgg tggctgttct     240
   cttttaagcg ggatagcatg ggttcttaga ggacccccta caaggattga ggattgttta     300
   atg aat tcc ccg cag aac gtc tcc acc aag aag gtc acc gtc acc ggc       348
   Met Asn Ser Pro Gln Asn Val Ser Thr Lys Lys Val Thr Val Thr Gly
   1               5                   10                  15
   gca gct ggt caa atc tct tat tca ctg ttg tgg cgc atc gct aac ggt       396
   Ala Ala Gly Gln Ile Ser Tyr Ser Leu Leu Trp Arg Ile Ala Asn Gly
               20                  25                  30
   gaa gta ttc ggc act gaa acc cct gtt gag ctg aag ctg ctg gaa atc       444
   Glu Val Phe Gly Thr Glu Thr Pro Val Glu Leu Lys Leu Leu Glu Ile
           35                  40                  45
   cct cag gca ctt ggt ggg gca gag ggc gtg gcc atg gaa ctt ctt gat       492
   Pro Gln Ala Leu Gly Gly Ala Glu Gly Val Ala Met Glu Leu Leu Asp
       50                  55                  60
   tca gcc ttc cca ctc ctg cga aac atc acc atc acc gcc gat gcc aat       540
   Ser Ala Phe Pro Leu Leu Arg Asn Ile Thr Ile Thr Ala Asp Ala Asn
   65                  70                  75                  80
   gag gca ttc gac ggc gct aat gcg gcg ttt ttg gtc ggt gcg aag cct      588
   Glu Ala Phe Asp Gly Ala Asn Ala Ala Phe Leu Val Gly Ala Lys Pro
                   85                  90                  95
   cgc gga aaa ggc gaa gag cgc gca gat ctg ctg gct aac aac ggc aag      636
   Arg Gly Lys Gly Glu Glu Arg Ala Asp Leu Leu Ala Asn Asn Gly Lys
               100                 105                 110
   att ttc gga cct caa ggt aaa gct atc aat gac aac gcc gca gat gac      684
   Ile Phe Gly Pro Gln Gly Lys Ala Ile Asn Asp Asn Ala Ala Asp Asp
           115                 120                 125
   att cgt gtc cta gtt gtt gga aac cca gcg aac acc aac gcg ttg att      732
   Ile Arg Val Leu Val Val Gly Asn Pro Ala Asn Thr Asn Ala Leu Ile
       130                 135                 140
   gct tca gct gcg gcc cca gat gtt cca gca tcc cgc ttc aac gca atg      780
   Ala Ser Ala Ala Ala Pro Asp Val Pro Ala Ser Arg Phe Asn Ala Met
   145                 150                 155                 160
   atg cgc ctt gat cac aac cgt gcg atc tcc cag ctg gcc acc aag ctt      828
   Met Arg Leu Asp His Asn Arg Ala Ile Ser Gln Leu Ala Thr Lys Leu
                   165                 170                 175
   ggc cgt gga tct gcg gaa ttt aac aac att gtg gtc tgg gga aat cac      876
   Gly Arg Gly Ser Ala Glu Phe Asn Asn Ile Val Val Trp Gly Asn His
               180                 185                 190
   tcc gca acc cag ttc cca gac atc acc tac gca acc gtt ggt gga gaa      924
   Ser Ala Thr Gln Phe Pro Asp Ile Thr Tyr Ala Thr Val Gly Gly Glu
           195                 200                 205
   aag gtc acc gac ctg gtt gat cac gat tgg tat gtg gag gag ttc att      972
   Lys Val Thr Asp Leu Val Asp His Asp Trp Tyr Val Glu Glu Phe Ile
       210                 215                 220
   cct cgc gtg gct aac cgt ggc gct gaa atc att gag gtc cgt gga aag     1020
   Pro Arg Val Ala Asn Arg Gly Ala Glu Ile Ile Glu Val Arg Gly Lys
   225                 230                 235                 240
   tct tct gca gct tct gca gca tcc tct gcg att gat cac atg cgc gat     1068
   Ser Ser Ala Ala Ser Ala Ala Ser Ser Ala Ile Asp His Met Arg Asp
                   245                 250                 255
   tgg gta cag ggc acc gag gcg tgg tcc tct gcg gca att cct tcc acc     1116
   Trp Val Gln Gly Thr Glu Ala Trp Ser Ser Ala Ala Ile Pro Ser Thr
               260                 265                 270
   ggt gca tac ggc att cct gag ggc att ttt gtc ggt ctg cca acc gta     1164
   Gly Ala Tyr Gly Ile Pro Glu Gly Ile Phe Val Gly Leu Pro Thr Val
           275                 280                 285
   tcc cgc aac ggt gag tgg gaa atc gtt gaa ggc ctg gag att tcc gat     1212
   Ser Arg Asn Gly Glu Trp Glu Ile Val Glu Gly Leu Glu Ile Ser Asp
       290                 295                 300
   ttc cag cgc gcc cgc atc gac gcg aat gct cag gaa ttg cag gcc gag     1260
   Phe Gln Arg Ala Arg Ile Asp Ala Asn Ala Gln Glu Leu Gln Ala Glu
   305                 310                 315                 320
   cgc gag gca gtg cgc gac ttg ctc taa tctttaacgc atgacttcgc           1307
   Arg Glu Ala Val Arg Asp Leu Leu
   325
   ttttcgacgc cccaaccctc caacgcgtca ccgttttcac gggctcggcg ctcggcagtt   1367
   cctcgctgta cacgcaagcg gctcaaacct tggcgaaaac cgcggtagac cgcggcatcg   1427
   acttggttta cggtggcgga aaagtggggc tcatgggtat cgtcgcggat gcgttcctgg   1487
   aatcaggtgg cgaag                                                    1502
<210> 14
   <211> 328
   <212> PRT
   <213> Brevibacterium flavum
<400> 14
   Met Asn Ser Pro Gln Asn Val Ser Thr Lys Lys Val Thr Val Thr Gly
   1               5                   10                  15
   Ala Ala Gly Gln Ile Ser Tyr Ser Leu Leu Trp Arg Ile Ala Asn Gly
               20                  25                  30
   Glu Val Phe Gly Thr Glu Thr Pro Val Glu Leu Lys Leu Leu Glu Ile
           35                  40                  45
   Pro Gln Ala Leu Gly Gly Ala Glu Gly Val Ala Met Glu Leu Leu Asp
       50                  55                  60
   Ser Ala Phe Pro Leu Leu Arg Asn Ile Thr Ile Thr Ala Asp Ala Asn
   65                  70                  75                  80
   Glu Ala Phe Asp Gly Ala Asn Ala Ala Phe Leu Val Gly Ala Lys Pro
                   85                  90                  95
   Arg Gly Lys Gly Glu Glu Arg Ala Asp Leu Leu Ala Asn Asn Gly Lys
               100                 105                 110
   Ile Phe Gly Pro Gln Gly Lys Ala Ile Asn Asp Asn Ala Ala Asp Asp
           115                 120                 125
   Ile Arg Val Leu Val Val Gly Asn Pro Ala Asn Thr Asn Ala Leu Ile
       130                 135                 140
   Ala Ser Ala Ala Ala Pro Asp Val Pro Ala Ser Arg Phe Asn Ala Met
   145                 150                 155                 160
   Met Arg Leu Asp His Asn Arg Ala Ile Ser Gln Leu Ala Thr Lys Leu
                   165                 170                 175
   Gly Arg Gly Ser Ala Glu Phe Asn Asn Ile Val Val Trp Gly Asn His
               180                 185                 190
   Ser Ala Thr Gln Phe Pro Asp Ile Thr Tyr Ala Thr Val Gly Gly Glu
           195                 200                 205
   Lys Val Thr Asp Leu Val Asp His Asp Trp Tyr Val Glu Glu Phe Ile
       210                 215                 220
   Pro Arg Val Ala Asn Arg Gly Ala Glu Ile Ile Glu Val Arg Gly Lys
   225                 230                 235                 240
   Ser Ser Ala Ala Ser Ala Ala Ser Ser Ala Ile Asp His Met Arg Asp
                   245                 250                 255
   Trp Val Gln Gly Thr Glu Ala Trp Ser Ser Ala Ala Ile Pro Ser Thr
               260                 265                 270
   Gly Ala Tyr Gly Ile Pro Glu Gly Ile Phe Val Gly Leu Pro Thr Val
           275                 280                 285
   Ser Arg Asn Gly Glu Trp Glu Ile Val Glu Gly Leu Glu Ile Ser Asp
       290                 295                 300
   Phe Gln Arg Ala Arg Ile Asp Ala Asn Ala Gln Glu Leu Gln Ala Glu
   305                 310                 315                 320
   Arg Glu Ala Val Arg Asp Leu Leu
                   325
<210> 15
   <211> 32
   <212> DNA
   <213> Artificial
<220>
   <223> primer
<900> 15
   gggaaggatc ccctttcggg taacgagaaa ac 32
<210> 16
   <211> 31
   <212> DNA
   <213> Artificial
<220>
   <223> primer
<400> 16
   gggaaggatc ccttcgccac ctgattccag g 31
<210> 17
   <211> 33
   <212> DNA
   <213> Artificial
<220>
   <223> primer
<400> 17
   gggcctgcag gaccaagacc gctgttgcag tga 33
<210> 18
   <211> 31
   <212> DNA
   <213> Artificial
<220>
   <223> primer
<400> 18
   gggcctgcag ggtattcaca ccagccccaa t 31
<210> 19
   <211> 40
   <212> DNA
   <213> Artificial
<220>
   <223> primer
<400> 19
   tcgcttttta ggagcacccc gtgagcttcc ttgtagaaaa 40
<210> 20
   <211> 20
   <212> DNA
   <213> Artificial
<220>
   <223> primer
<400> 20
   ccaaggtcag tgcgttgttg 20
<210> 21
   <211> 29
   <212> DNA
   <213> Artificial
<220>
   <223> primer
<400> 21
   ggtctagaag ttcgccgacg gaatccacg 29
<210> 22
   <211> 40
   <212> DNA
   <213> Artificial
<220>
   <223> primer
<400> 22
   ttttctacaa ggaagctcac ggggtgctcc taaaaagcga 40
<210> 23
   <211> 27
   <212> DNA
   <213> Artificial
<220>
   <223> primer
<400> 23
   ggtctagacg atcaattcct ttcgggt 27
<210> 24
   <211> 30
   <212> DNA
   <213> Artificial
<220>
   <223> primer
<400> 24
   ggcctgcagg gcgttaaaga ttagagcaag 30
<210> 25
   <211> 40
   <212> DNA
   <213> Artificial
<220>
   <223> primer
<400> 25
   gaagtccagg aggacataca atgaattccc cgcagaacgt 40
<210> 26
   <211> 20
   <212> DNA
   <213> Artificial
<220>
   <223> primer
<400> 26
   tcattgcgtt gaagcgggat 20
<210> 27
   <211> 29
   <212> DNA
   <213> Artificial
<220>
   <223> primer
<400> 27
   ggggatccag atcgtttaga tccgaagga 29
<210> 28
   <211> 40
   <212> DNA
   <213> Artificial
<220>
   <223> primer
<400> 28
   acgttctgcg gggaattcat tgtatgtcct cctggacttc 40
<210> 29
   <211> 30
   <212> DNA
   <213> Artificial
<220>
   <223> primer
<400> 29
   ggcctgcagg gcgttaaaga ttagagcaag 30
<210> 30
   <211> 28
   <212> DNA
   <213> Artificial
<220>
   <223> primer
<400> 30
   ggactagtag atcgtttaga tccgaagg 28
<210> 31
   <211> 20
   <212> DNA
   <213> Artificial
<220>
   <223> primer
<400> 31
   cactgcacgg ccctgcgaac 20
<210> 32
   <211> 42
   <212> DNA
   <213> Artificial
<220>
   <223> primer
<400> 32
   cgccaactag gcgccaaaaa ttcctgattt ccctaaccgg ac 42
<210> 33
   <211> 42
   <212> DNA
   <213> Artificial
<220>
   <223> primer
<400> 33
   gtccggttag ggaaatcagg aatttttggc gcctagttgg cg 42
<210> 34
   <211> 20
   <212> DNA
   <213> Artificial
<220>
   <223> primer
<400> 34
   tgtgggcctt cggcgaggac 20
<210> 35
   <211> 26
   <212> DNA
   <213> Artificial
<220>
   <223> primer
<400> 35
   gagtcgaccg caccccattt ttcata 26
<210> 36
   <211> 28
   <212> DNA
   <213> Artificial
<220>
   <223> primer
<400> 36
   tggtcgacgt gaatgctcgg cgggatcc 28
<210> 37
   <211> 300
   <212> DNA
   <213> Corynebacterium glutamicum
<400> 37
   aagcgcctca tcagcggtaa ccatcacggg ttcgggtgcg aaaaaccatg ccataacagg     60
   aatgttcctt tcgaaaattg aggaagcctt atgcccttca accctactta gctgccaatt    120
   attccgggct tgtgacccgc tacccgataa ataggtcggc tgaaaaattt cgttgcaata    180
   tcaacaaaaa ggcctatcat tgggaggtgt cgcaccaagt acttttgcga agcgccatct    240
   gacggatttt caaaagatgt atatgctcgg tgcggaaacc tacgaaagga ttttttaccc     300
<210> 38
   <211> 299
   <212> DNA
   <213> Corynebacterium glutamicum
<400> 38
   gcggcccggg ttggcggcct gcccatcgca gtcgcggaag gggagacggg attgcttgtc     60
   gacggccact ccccgcatgc ctgggccgac gccttagcca cactcttgga cgatgacgaa    120
   acgcgcatca gaatgggtga agacgccgtc gaacacgcca gaacattctc ctgggcggcc    180
   accgccgcac agctatcgtc gctgtacaac gacgctattg ccaacgaaaa tgtcgacggt    240
   gaaacgcatc acggctaagt aaacgcgcgt cgtggaacat aaagtggcaa actagtacc     299
<210> 39
   <211> 285
   <212> DNA
   <213> Corynebacterium glutamicum
<900> 39
<210> 40
   <211> 300
   <212> DNA
   <213> Corynebacterium glutamicum
<400> 40
   atcgacggca tgaaaccttg cacaaaagtg ctataggttt cgttgatcaa ccggcgtgat      60
   tccgcagcaa ttgtgccgaa caccagcgag gacttgccag atcccgacac acccgtgaac     120
   acggtgagac gccttttagg gatgcgcacc gacacatttt tgaggttgtt ttcattcgca     180
   ccgtggaccg aaatccaatc atgggaatca gctttttgca tgtgtcatat cgtaccgttt     240
   gcataggcct gttcgcgctt ggtgaacctt ttctagcacc aaaacaaaac tctccctagt     300
<210> 41
   <211> 300
   <212> DNA
   <213> Corynebacterium glutamicum
<400> 41
   gatagttcaa accgccaggc aacttcactg agattccacc acgcgaatta atagtcaccg      60
   gcccgattcg cttcgatccg gaaacgccag acttagaaac attcagccaa ctgtcttttc     120
   cggtcttaat aattttccga taattcagtc ccatgtgaac cagcataatc ttcatcacat     180
   ggtgtgtgca gaaatatttt tgctggtcta ttgtggcgac cgagggcctt tgaaggttcg     240
   acaaactgta taaggccttg aatcttgaga atttattttg aggaagcaag aggaagtgtc     300
<210> 42
   <211> 300
   <212> DNA
   <213> Corynebacterium glutamicum
<400> 42
   actatttagc gcaagtgttg gaaatgcccc cgtttggggt caatgtccat ttttgaatgt      60
   gtctgtatga ttttgcatct gctgcgaaat ctttgtttcc ccgctaaagt tgaggacagg     120
   ttgacacgga gttgactcga cgaattatcc aatgtgagta ggtttggtgc gtgagttgga     180
   aaaattcgcc atactcgccc ttgggttctg tcagctcaag aattcttgag tgaccgatgc     240
   tctgattgac ctaactgctt gacacattgc atttcctaca atctttagag gagacacaac     300
<210> 43
   <211> 300
   <212> DNA
   <213> Corynebacterium glutamicum
<400> 43
   tcgccgcggg tgaggtggcc ttcggatgcg atgagggctg cgtcgatgtt gatttctgaa      60
   aaatcgttga caatgactgc gattttccgg gatcctcggt ggtggaggat ctggttgagc     120
   aatgtggttt ttccggagcc gagaaatcct gatagcacgg tgactggggt cgttgtggcc     180
   atgtgaaagc cctccttttg ggaatcattt tcaatagagt cgacgcaagt gtacacttct     240
   taatggaaat tgttttcaat aaagtcaagt tttttgacct tcgcttttta ggagcacccc     300
<210> 44
   <211> 300
   <212> DNA
   <213> Corynebacterium glutamicum
<900> 44
<210> 45
   <211> 300
   <212> DNA
   <213> Brevibacgterium flavum
<400> 45
   gcgttttagc gtgtcagtag gcgcgtaggg taagtggggt agcggcttgt tagatatctt     60
   gaaatcggct ttcaacagca ttgatttcga tgtatttagc tggccgttac cctgcgaatg    120
   tccacagggt agctggtagt ttgaaaatca acgccgttgc ccttaggatt cagtaactgg    180
   cacattttgt aatgcgctag atctgtgtgc tcagtcttcc aggctgctga tcacagtgaa    240
   agcaaaacca attcgtggct gcgaaagtcg tagccaccac gaagtccagg aggacataca    300